(19) Europäisches Patentamt
European Patent Office
Office européen des brevets

(11) EP 0 569 333 B1

(12) # EUROPÄISCHE PATENTSCHRIFT

(45) Veröffentlichungstag und Bekanntmachung des Hinweises auf die Patenterteilung:
**07.01.1999 Patentblatt 1999/01**

(51) Int Cl.[6]: **C07F 9/30**, A61K 31/66, C07F 9/653, C07F 9/58, C07F 9/6518, C07F 9/6524

(21) Anmeldenummer: **93810316.5**

(22) Anmeldetag: **29.04.1993**

(54) **Neue N-Aralkyl- und N-Heteroaralkylaminoalkanphosphinsäuren**

Novel N-aralkyl and N-heteroaralkylamino alcane phosphinic acids

Nouveaux acides N-aralkyl et N-hétéroaralkylamino alcane phosphoniques

(84) Benannte Vertragsstaaten:
**AT BE CH DE DK ES FR GB GR IE IT LI LU NL PT SE**

(30) Priorität: **08.05.1992 CH 1480/92**

(43) Veröffentlichungstag der Anmeldung:
**10.11.1993 Patentblatt 1993/45**

(73) Patentinhaber: **Novartis AG**
**4058 Basel (CH)**

(72) Erfinder:
- **Mickel, Stuart John, Dr.**
  **CH-4415 Lausen (CH)**
- **Fröstl, Wolfgang, Dr.**
  **CH-4056 Basel (CH)**
- **Furet, Pascal, dr.**
  **F-68800 Thann (FR)**

(56) Entgegenhaltungen:
**EP-A- 0 319 482**
**EP-A- 0 463 560**
**EP-A- 0 543 780**
**WO-A-93/11138**

EP 0 569 333 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue N-Aralkyl- und N-Heteroaralkylaminoalkanphosphinsäuren der Formel I

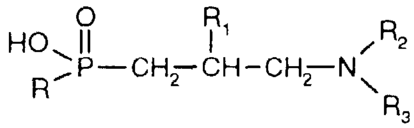

(I),

worin R $C_3$-$C_7$-Alkyl, α,α-Di-$C_1$-$C_4$-alkoxy-$C_1$-$C_4$-alkyl, $C_3$-$C_6$-Cycloalkyl-$C_1$-$C_4$-alkyl, $C_3$-$C_6$-Cycloalkenyl-$C_1$-$C_4$-alkyl, oder unsubstituiertes oder durch $C_1$-$C_4$-Alkyl, $C_1$-$C_4$-Alkoxy, Hydroxy und/oder Halogen, mono-, di- oder trisubstituiertes Phenyl-$C_1$-$C_4$-alkyl bedeutet, $R_1$ Wasserstoff oder Hydroxy darstellt, $R_2$ einen durch Carboxy, $C_1$-$C_4$-Alkoxycarbonyl, Cyano, Carbamoyl, N-Mono- oder N,N-Di-$C_1$-$C_4$-alkylcarbamoyl, Carboxy-$C_1$-$C_4$-alkyl, $C_1$-$C_4$-Alkoxycarbonyl-$C_1$-$C_4$-alkyl, Cyano-$C_1$-$C_4$-alkyl, Carbamoyl-$C_1$-$C_4$-alkyl, N-Mono- oder N,N-Di-$C_1$-$C_4$-alkylcarbamoyl-$C_1$-$C_4$-alkyl, unsubstituiertes oder durch $C_1$-$C_4$-Alkyl, $C_1$-$C_4$-Alkoxy, $C_1$-$C_4$. Alkoxycarbonyl, Cyano, Hydroxy, Amino oder Halogen substituiertes Oxazolyl, Isoxazolyl, Oxadiazolyl, Triazolyl, Tetrazolyl, Oxazolyl-$C_1$-$C_4$-alkyl, Isoxazolyl-$C_1$-$C_4$-alkyl, Oxadiazolyl-$C_1$-$C_4$-alkyl, Triazolyl-$C_1$-$C_4$-alkyl oder Tetrazolyl-$C_1$-$C_4$-alkyl ein- oder zweifach und gegebenenfalls zusätzlich durch $C_1$-$C_4$-Alkoxy, Polyfluor-$C_1$-$C_4$-alkoxy, Halogen oder Polyfluor-$C_1$-$C_4$-alkyl substituierten Phenyl-, Thienyl-, Furyl- oder Pyridyl-$C_1$-$C_4$-alkylrest und $R_3$ Wasserstoff oder $C_1$-$C_4$-Alkyl bedeutet, und ihre Salze, Verfahren zu ihrer Herstellung, diese enthaltende pharmazeutische Präparate und ihre Verwendung als Arzneimittelwirkstoffe.

Oxazolyl ist z.B. 5-$R_a$-Oxazol-2-yl,Isoxazolyl z.B.3-$R_a$-Isoxazol-5-yl, Oxadiazolyl z. B. 3-$R_a$-1,2,4-Oxadiazol-5-yl, 5-$R_a$-1,2,4-Oxadiazol-3-yl oder 5-$R_a$-1,3,4-Oxadiazol-3-yl, Triazolyl z.B.5-$R_a$-1,2,4-Triazol-3-yl oder 3-$R_a$-1,2,4-Triazol-5-yl und Tetrazolyl, z.B.Tetrazol-5-yl, wobei $R_a$ Wasserstoff oder in zweiter Linie $C_1$-$C_4$-Alkyl, $N_1$-$C_4$-Alkoxy, $C_1$-$C_4$-Alkoxycarbonyl, Hydroxy, Amino oder Halogen bedeutet, vorzugsweise unsubstituiertes oder in 3-Stellung durch Amino oder Halogen, wie Chlor, substituiertes 1,2,4-Oxadiazol-5-yl, in Betracht.

$C_3$-$C_7$-Alkyl R ist vorzugsweise $C_3$-$C_5$-Alkyl, wie Propyl, Isopropyl, Butyl, Isobutyl, Sekundärbutyl, Tertiärbutyl oder Pentyl.

$C_1$-$C_4$-Alkyl $R_3$ ist beispielsweise Methyl, Ethyl, Propyl, Isopropyl oder Butyl, kann aber auch Isobutyl, Sekundärbutyl, Tertiärbutyl.

Phenyl-$C_1$-$C_4$-alkyl ist beispielsweise Benzyl, 1-Phenylethyl, 2-Phenylprop-2-yl oder in zweiter Linie 2-Phenylethyl, 2-Phenylprop-1-yl oder 3-Phenylprop-1-yl.

Thienyl-, Furyl- oder Pyridyl-$C_1$-$C_4$-alkyl ist beispielsweise Thienyl-, Furyl- oder Pyridylmethyl, 1-Thienyl-, 1-Furyl- oder 1-Pyridylethyl, 2-Thienyl-, 2-Furyl- oder 2-Pyridylprop-2-yl, oder in zweiter Linie 2-Thienyl-, 2-Furyl- oder 2-Pyridylethyl, 2-Thienyl-, 2-Furyl- oder 2-Pyridylprop-1-yl oder 3-Thienyl-, 3-Furyl- oder 3-Pyridylprop-1-yl.

Polyfluor-$C_1$-$C_4$-alkyl ist beispielsweise - Di- oder Trifluor-$C_2$-$C_5$-alkyl, wie 3,3,3-Trifluorpropyl, 4,4,4-Trifluorbutyl, 1- oder 2-Fluorbutyl oder 1,1-Difluorbutyl.

$C_1$-$C_4$-Alkoxy ist beispielsweise Methoxy, Ethoxy, Propyloxy, Isopropyloxy oder Butyloxy, kann aber auch Isobutyloxy, Sekundärbutyloxy oder Tertiärbutyloxy.

$C_1$-$C_4$-Alkoxycarbonyl ist beispielsweise Methoxy-, Ethoxy-, Propyloxy-, Isopropyloxy- oder Butyloxycarbonyl, kann aber auch Isobutyloxycarbonyl, Sekundärbutyloxycarbonyl oder Tertiärbutyloxycarbonyl sein.

N-Mono- oder N,N-Di-$C_1$-$C_4$-alkylcarbamoyl ist beispielsweise Methyl-, Ethyl-, Dimethyl- oder Diethylcarbamoyl.

Carboxy-$C_1$-$C_4$-alkyl ist beispielsweise Carboxymethyl.

$C_1$-$C_4$-Alkoxycarbonyl-$C_1$-$C_4$-alkyl ist beispielsweise Methoxy- oder Ethoxycarbonylmethyl.

Cyano-$C_1$-$C_4$-alkyl ist beispielsweise Cyanomethyl.

Carbamoyl- bzw. N-Mono- oder N,N-Di-$C_1$-$C_4$-alkylcarbamoyl-$C_1$-$C_4$-alkyl ist beispielsweise Carbamoylmethyl oder Methyl-, Ethyl-, Dimethyl- oder Diethylcarbamoylmethyl

Polyfluor-$C_1$-$C_4$-alkoxy ist beispielsweise Trifluor-$C_1$-$C_4$-alkoxy, wie Trifluormethoxy.

Di-$C_1$-$C_4$-alkoxy-$C_1$-$C_4$-alkyl ist beispielsweise Dimethoxymethyl, Dipropyloxymethyl, 1,1- oder 2,2-Diethoxyethyl, Diisopropyloxymethyl, Dibutyloxymethyl oder 3,3-Dimethoxypropyl.

Halogen ist beispielsweise Halogen der Atomnummer bis und mit 35, wie Chlor oder Fluor, femer Brom.

$C_3$-$C_6$-Cycloalkyl ist beispielsweise Cyclopropyl, Cyclobutyl, Cyclopentyl oder Cyclohexyl.

$C_3$-$C_6$-Cycloalkyl-$C_1$-$C_4$-alkyl ist beispielsweise α-($C_3$-$C_6$-Cycloalkyl)-$C_1$-$C_4$-alkyl, z.B. Cyclopropylmethyl, Cyclobutylmethyl, Cyclopentylmethyl oder Cyclohexylmethyl.

$C_3$-$C_6$-Cycloalkenyl-$C_1$-$C_4$-alkyl ist beispielsweise α-($C_3$-$C_6$-Cycloalkenyl)-$C_1$-$C_4$-alkyl, z.B. Cyclopent-1-enylmethyl, Cyclopent-2-enylmethyl, Cyclopent-3-enylmethyl, Cyclohex-1-enylmethyl, Cyclohex-2-enylmethyl oder Cyclohexyl-3-enylmethyl.

Die Verbindungen der Formel I liegen auf Grund ihres amphoteren Charakters in Form innerer Salze vor und

können sowohl Säureadditionssalze als auch Salze mit Basen bilden.

Säureadditionssalze von Verbindungen der Formel I sind beispielsweise deren pharmazeutisch verwendbare Salze mit geeigneten Mineralsäuren, wie Halogenwasserstoffsäuren Schwefelsäure oder Phosphorsäure z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Salze von Verbindungen der Formel I mit Basen sind beispielsweise deren Salze mit pharmazeutisch verwendbaren Basen, wie nicht-toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, ebenso Ammoniumsalze mit Ammoniak oder organischen Aminen oder quatemären Ammoniumbasen, wie gegebenenfalls C-hydroxylierten aliphatischen Aminen, insbesondere Mono-, Di- oder Triniederalkylaminen, z.B. Methyl-, Ethyl- oder Diethylamin, Mono-, Di- oder Tri-(hydroxyniederalkyl)aminen, wie Ethanol-, Diethanol- oder Triethanolamin, Tris-(hydroxymethyl)methylamin oder 2-Hydroxytertiärbutylamin, oder N-(Hydroxyniederalkyl)-N, N-diniederalkylaminen bzw. N-(Polyhydroxyniederalkyl)-niederalkylaminen, wie 2-(Dimethylamino)ethanol oder D-Glucamin, oder quaternären aliphatischen Ammoniumhydroxiden, z.B. Tetrabutylammoniumhydroxid.

Abhängig vom Vorhandensein asymmetrischer Kohlenstoffatome können die erfindungsgemässen Verbindungen in Form von Isomerengemischen, speziell als Racemate, oder in Form reiner Isomere, speziell von optischen Antipoden vorliegen.

In EP-A-0 463 560 und EP-A-0 543 780 sind Verbindungen der Formel I beschrieben, die als Gruppe $R_2$ einen im Phenyl- bzw. Naphthylteil durch $C_1$-$C_4$-Alkyl, $C_1$-$C_4$-Alkoxy, Hydroxy und/oder Halogen mono-, di- oder trisubstituierten Phenyl-$C_1$-$C_4$-alkyl-, Diphenyl-$C_1$-$C_4$-alkyl- oder Naphthyl-$C_1$-$C_4$-alkylrest oder einen unsubstituierten oder im Thienyl-, Furyl- bzw. Pyridylteil durch Halogen substituierten Thienyl-, Furyl- oder Pyridyl-$C_1$-$C_4$-alkylrest aufweisen.

Es wurde nun gefunden, dass die erfindungsgemäss bereitgestellten Verbindung der Formel I und ihre pharmazeutisch verwendbaren Salze wertvolle pharmakologische Eigenschaften besitzen. Sie zeigen eine wirksame Bindung an den $GABA_B$-Rezeptor und erwiesen sich als Antagonisten von GABA ($\gamma$-aminobutyric acid) an diesem Rezeptor. Mechanistisch gesehen kann Antagonismus an $GABA_B$-Rezeptoren die Freisetzung von schnellen Reiz-Aminosäuren--Transmittem, d.h. Glutamat und Aspargat, steigem und so die Informationsverarbeitung im Gehim verbessem. Damit stimmt die Erkenntnis überein, dass das späte postsynaptische Inhibitionspotential im Hippocampus, das einem $GABA_B$-Mechanismus zugeschrieben wird, durch die Antagonisten abgebaut wird und damit eine schnellere Nervenimpulsübertragungsfolge ermöglicht.

Andererseits wurde gefunden, dass die chronische Behandlung mit Antidepressiva und wiederholte Elektroschocks die Zahl der $GABA_B$-Rezeptoren in der Hirnrinde bei Ratten erhöht. In Übereinstimmung mit Rezeptortheorien sollte die chronische Behandlung mit $GABA_B$-Antagonisten zu derselben Wirkung führen. Aus diesem und anderen Gründen können daher $GABA_B$-Antagonisten als Antidepressiva wirken.

Die erfindungsgemässen $GABA_B$-Antagonisten wechselwirken am $GABA_B$-Rezeptor mit $IC_{50}$-Werten ab etwa $10^{-8}$M (Mol/l) an Rattenhirnrindenmembranen. Im Gegensatz zu $GABA_B$-Agonisten wie Baclofen potenzieren sie nicht die Stimulierung von Adenylatcyclase an Rattenhimrindenschnitten durch Noradrenalin, sondem wirken als Antagonist der Baclofen-Wirkung. Der Antagonismus gegenüber Baclofen wurde auch an elektrophysiologischen Modellen *in vitro* gezeigt, wie z.B. am Penicillin-induzierten "epileptischen" Hippocampus-Schnittpräparat, wo Baclofen bei einer Konzentration von 6$\mu$M (Mikromol/Liter) "epilepsieartige" Entladungen von Pyramidenzellen inhibiert. Die erfindungsgemässen Verbindungen wirken als Antagonist der Baclofen-Wirkung bei Konzentrationen von etwa 1 bis etwa 100nM (Nanomol/Liter). *In vivo* konnte der Antagonismus durch Iontophorese von Baclofen an Rattenhirnrinde und durch systemische Anwendung von Antagonisten in Dosen von etwa 1 bis etwa 100mg/kg bewiesen werden. Bei Dosen von etwa 10 bis etwa 30mg/kg tritt Antagonismus gegen die muskelrelaxierende Wirkung von Baclofen ein, die im Rotarod-Modell gemessen wird.

Die Antagonisten zeigen nicht nur Antagonismus gegen Baclofen, sondern weisen auch eine selbständige Wirkung als Antagonisten von endogenem GABA auf. Somit sind die Antagonisten aktiv bei herkömmlichen Verhaltensmodellen, die charakteristisch sind für antidepressive, anxiolytische und/oder nootrope Eigenschaften. Verbindungen der Formel (I), so wurde gefunden, sind bei oraler Anwendung aktiv im Schwimmtest nach Porsolt, im Geller-Test, dem verzögerten passiven Meidungstest (Einversuchs-Modifizierung) in Vorversuchs- und Nachversuchs-Situationen, im Zweikammertest und im komplexen Labyrinth. Ausserdem wurde bei Untersuchungen an Rhesusaffen ein gesteigerter Spieltrieb, Neugierde, soziales Pflegeverhalten und eine Verminderung von Angstmerkmalen beobachtet.

Ferner wurde festgestellt, dass die Verbindungen der Formel I und ihre pharmazeutisch verwendbaren Salze, *in vivo* antiepileptische Eigenschaft, insbesondere eine ausgeprägte Antiabsensenz-Wirkung bei epileptischen Erkrankungen des "petit mal"- Formenkreises, d.h. der Absenzepilepsie bei Kindem und Jugendlichen sowie von atypischen Absenzen, wie des Lennox-Gastaut-Syndroms, aufweisen.

Diese kann an einem bestimmten Rattenstamm anhand ihrer ausgeprägten Hemmwirkung auf spontane "Spike and Wave"-Entladungen im Tiermodell gemäss Vergnes M., Marescaux C., Micheletti G., Reis J., Depaulis A., Rumbach

L and Warter J.M., Neurosci. Lett. 33, 97-101 (1982) gezeigt werden.

Die Verbindungen der Formel I und ihre pharmazeutisch verwendbaren Salze sind deshalb vorzüglich geeignet als Nootropika, Antidepressiva und Anxiolytika, z.B. zur Behandlung von cerebralen Insuffizienzerscheinungen, Gemütsdepressionen, Angstzuständen und von Epilepsien des "petit mal"-Formenkreises, d.h. der Absenzepilepsie bei Kindern und Jugendlichen sowie von atypischen Absenzen, wie des Lennox-Gastaut-Syndroms, sowie als Baclofen-Gegenmittel.

Die Erfindung betrifft insbesondere Verbindungen der Formel I, worin R $C_3$-$C_5$-Alkyl, wie Butyl, $\alpha,\alpha$-Di-$C_1$-$C_4$-alkoxymethyl, wie Diethoxymethyl, $C_3$-$C_6$-Cycloalkyl-$C_1$-$C_4$-alkyl, wie Cyclopropyl- oder Cyclohexylmethyl, oder Benzyl bedeutet, $R_1$ Wasserstoff oder Hydroxy darstellt, $R_2$ einen durch Carboxy, $C_1$-$C_4$-Alkoxycarbonyl, wie Methoxy- oder Ethoxycarbonyl, Cyano, Carbamoyl, Carboxy-$C_1$-$C_4$-alkyl, wie Carboxymethyl, $C_1$-$C_4$-Alkoxycarbonyl-$C_1$-$C_4$-alkyl, wie Methoxy- oder Ethoxycarbonylmethyl, Cyano-$C_1$-$C_4$-alkyl, wie Cyanomethyl, Carbamoyl-$C_1$-$C_4$-alkyl, wie Carbamoylmethyl, Isoxazol-5-yl, Isoxazo-2-yl, 1,2,4-Oxadiazol-5-yl, 1,2,4-Oxadiazol-3-yl, 1,3,4-Oxadiazol-3-yl, 1,2,4-Triazol-3-yl, 1,2,4-Triazol-5-yl oder Tetrazol-5-yl, Isoxazol-5-ylmethyl, Isoxazol-2-ylmethyl, 1,2,4-Oxadiazol-5-ylmethyl, 1,2,4-Oxadiazol-3-ylmethyl, 1,3,4-Oxadiazol-3-ylmethyl, 1,2,4-Triazol-3-ylmethyl, 1,2,4-Triazol-5-ylmethyl oder Tetrazol-5-ylmethyl, ein- oder zweifach und gegebenenfalls zusätzlich durch $C_1$-$C_4$-Alkoxy, wie Methoxy, Trifluormethoxy, Halogen, wie Fluor, Chlor oder Brom, oder Trifluormethyl substituierten $\alpha$-Phenyl-$C_1$-$C_4$-alkylrest, wie Benzyl-, 1-Phenylethyl- oder 2-Phenylprop-2-ylrest, oder $\alpha$-Pyridyl-$C_1$-$C_4$-alkyl-, wie Pyridylmethylrest, darstellt und $R_3$ Wasserstoff bedeutet, und ihre Salze, insbesondere ihre pharmazeutisch verwendbaren Salze.

Die Erfindung betrifft vorzugsweise Verbindungen der Formel 1, wonn R $\alpha,\alpha$-Di--$C_1$-$C_4$-alkoxymethyl, wie Diethoxymethyl, $C_3$-$C_6$-Cycloalkyl-$C_1$-$C_4$-alkyl, wie Cyclohexylmethyl, $C_3$-$C_6$-Cycloalk-3-enyl-$C_1$-$C_4$-alkyl, wie Cyclohex-3-enylmethyl, oder Benzyl bedeutet, $R_1$ Hydroxy darstellt, $R_2$ einen durch Carboxy, Cyano oder unsubstituiertes oder durch Amino oder Halogen substituiertes 1,2,4-Oxadiazol-5-yl substituierten Phenyl-$C_1$-$C_4$-alkyl-, wie Benzyl-, 1-Phenylethyl- oder 2-Phenylprop-2-ylrest darstellt und $R_3$ Wasserstoff bedeutet, und ihre Salze, insbesondere ihre pharmazeutisch verwendbaren Salze.

Die Erfindung betrifft in allererster Linie Verbindungen der Formel I, worin R $C_3$-$C_5$-Alkyl, wie Butyl, $\alpha,\alpha$-Di-$C_1$-$C_4$-alkoxymethyl, wie Diethoxymethyl, $C_3$-$C_6$-Cycloalkyl-$C_1$-$C_4$-alkyl, wie Cyclopropyl- oder Cyclohexylmethyl, oder Benzyl bedeutet, $R_1$ Wasserstoff oder Hydroxy darstellt, $R_2$ einen durch Carboxy, $C_1$-$C_4$-Alkoxycarbonyl, wie Methoxy- oder Ethoxycarbonyl, Cyano oder Carbamoyl ein- oder zweifach und gegebenenfalls zusätzlich durch $C_1$-$C_4$-Alkoxy, wie Methoxy, Trifluormethoxy, Halogen, wie Fluor, Chlor oder Brom, oder Trifluormethyl substituierten Phenyl-$C_1$-$C_4$-alkyl-, wie Benzyl-, 1 -Phenylethyl- oder 2-Phenylprop-2-ylrest darstellt und $R_3$ Wasserstoff bedeutet, und ihre Salze, insbesondere ihre pharmazeutisch verwendbaren Salze.

Die Erfindung betrifft jeweils vorzugsweise Verbindungen der Formel I, in denen das mit der Gruppe $R_1$ verbundene C-Atom der Propylenkette, sofern $R_1$ Hydroxy ist, und/oder ein chirales $\alpha$-C-Atom des aliphatischen Teils des Restes $R_2$, sofern ein solches vorhanden ist, jeweils (S)-Konfiguration aufweisen.

Die Erfindung betrifft namentlich die in den Beispielen genannten Verbindungen und ihre Salze, insbesondere Ihre pharmazeutisch verwendbaren Salze.

Das Verfahren zur Herstellung der erfindungsgemäss bereitgestellten Verbindungen der Formel I ist dadurch gekennzeichnet, dass man in einer Verbindung der Formel II

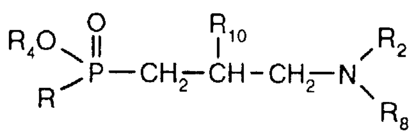

(II),

worin $R_4$ für eine Hydroxyschutzgruppe, $R_8$ für eine Gruppe $R_3$ oder für eine Aminoschutzgruppe und $R_{10}$ für Wasserstoff oder geschütztes Hydroxy steht, wobei R, $R_1$, $R_2$ und $R_3$ die angegebene Bedeutung haben, oder in einem Salz davon die Hydroxygruppen durch Ersatz der Hydroxyschutzgruppe $R_4$ durch Wasserstoff freisetzt und gegebenenfalls die Aminoschutzgruppe $R_8$ abspaltet und gegebenenfalls die geschützte Hydroxygruppen aus $R_{10}$ freisetzt und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

Geschützte Hydroxygruppen $R_4O$, ebenso wie $R_{10}$, sind beispielsweise veretherte Hydroxygruppen, vorzugsweise mit einem aliphatischen, cycloaliphatischen, araliphatischen oder aromatischen Alkohol oder mit einem Silanol veretherte Hydroxygruppen, insbesondere Niederalkoxy, Niederalkenyloxy, gegebenenfalls, beispielsweise durch Niederalkyl, Niederalkoxy, Halogen und/oder Nitro substituiertes Phenyloxy oder Phenylalkoxy, wie Benzyloxy, oder Triniederalkylsilyloxy, wie Trimethylsilyloxy, Tributylsilyloxy oder Tertiärbutyl(dimethyl)silyloxy.

Der Ersatz der Schutzgruppe $R_4$ in den Verbindungen der Formel I durch Wasserstoff kann erfolgen durch Behandlung mit einem geeigneten basischen oder sauren Mittel wie einem Alkalimetallhydroxid, z.B. Natriumhydroxid oder Lithiumhydroxid, einem Alkalimetallhalogenid, insbesondere -bromid oder -jodid wie Lithiumbromid oder Natriumjodid, Thioharnstoff, einem Alkalimetallthiophenolat wie Natriumthiophenolat oder einer protonischen oder Lewis-Säure wie einer Mineralsäure, z.B. Salzsäure oder einem Tri-Niederalkylhalosilan, z.B. Trimethylchlorsilan. Die Austauschreaktion kann in Abwesenheit oder Gegenwart eines Lösungsmittels und erforderlichenfalls unter Erhitzen oder unter Kühlung in einem geschlossenen Gefäss und/oder unter Inertgasatmosphäre durchgeführt werden.

Andererseits kann auch der Ersatz der $R_4$-Schutzgruppe, z.B. einer Silyl- oder Alkylgruppe, in Verbindungen der Formel I durch Wasserstoff oder durch Behandeln mit einer Säure unter hydrolytischen Bedingungen bewirkt werden, insbesondere mit einer Mineralsäure wie einer Halogenwasserstoffsäure, z.B. Salzsäure, die in verdünnter oder konzentrierter wässriger Form verwendet wird, oder durch Behandlung mit einem organischen Silylhalogenid, wie Trimethyljodsilan oder Trimethylbomsilan und erforderlichenfalls anschliessende Hydrolyse. Die Reaktion wird vorzugsweise bei erhöhter Temperatur durchgeführt, z.B. durch Halten des Reaktionsgemisches am Rückfluss und gegebenenfalls unter Verwendung eines organischen Verdünnungsmittels in einem geschlossenen Gefäss und/oder unter einer Inertgasatmosphäre. Die Art und Weise des Ersatzes der Schutzgruppe $R_4$ hängt beispielsweise von dem Substituenten R ab, der in der Verbindung der Formel I enthalten ist und bei der Umwandlung der Verbindung II in eine Verbindung der Formel I erhalten bleiben muss. Die genannte Umwandlung kann z.B. wie in den erläutemden Beispielen erfolgen.

Aminoschutzgruppen $R_8$ in Verbindungen der Formel I können nach bekannten Verfahren, die je nach der Art der Aminoschutzgruppe ausgewählt werden, abgespalten werden, z.B. durch solvolytische oder hydrogenolytische Verfahren, z.B. Hydrolyse in Gegenwart einer Säure oder Base, Acidolyse, z.B. Behandlung mit Trifluoressigsäure, Behandlung mit Hydrazin oder Hydrogenolyse in Gegenwart eines metallischen Hydrierungskatalysators, oder durch irgendein anderes geeignetes Verfahren.

Je nach den beteiligten Gruppen kann der Austausch und die Umwandlung nacheinander oder gleichzeitig nach an sich bekannten Methoden erfolgen.

Vorzugsweise werden alle Schutzgruppen, Hydroxy-Schutzgruppen $R_4$ bzw. $R_{10}$, und Aminoschutzgruppen $R_8$ in einer einzigen Stufe durch Behandlung mit einer Säure, vorzugsweise mit einer Halogenwasserstoffsäure, speziell Salzsäure unter hydrolytischen Bedingungen, durch Wasserstoff ersetzt.

Die Ausgangsstoffe der Formel II können auf verschiedenen Wegen hergestellt werden, beispielsweise indem man

a) in eine Verbindung der Formel III

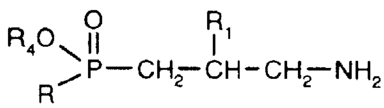

(III)

die Gruppe $R_2$ und gewünschtenfalls einen von Wasserstoff verschiedenen Rest $R_3$ einführt oder

b) eine Verbindung der Formel IV

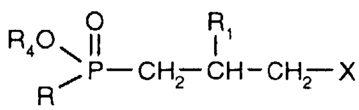

(IV),

worin X eine reaktionsfähige veresterte Hydroxygruppen bedeutet, oder ein Salz davon mit einem Amin der Formel V

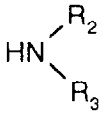

(V)

umsetzt oder

c) eine Verbindung der Formel VI

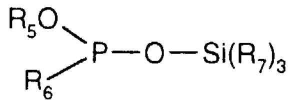

(VI),

worin $R_5$ eine Gruppe $R_4$ oder -$Si(R_7)_3$, $R_6$ einen an einer gegebenenfalls vorhandenen Hydroxygruppe durch eine Gruppe -$Si(R_7)_3$ geschützten Rest R und die Reste $R_7$ gleiche oder verschiedene aliphatische Kohlenwasserstoffreste, beispielsweise Niederalkyl, insbesondere
Methyl und/oder Tertiärbutyl, bedeuten, mit einer Verbindung der Formel VII

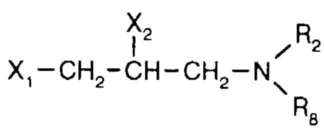

(VII),

worin $X_1$ reaktionsfähige verestertes Hydroxy und $X_2$ Wasserstoff bedeutet oder $X_1$ und $X_2$ gemeinsam für Epoxy stehen und $R_8$ eine Gruppe $R_3$ oder eine Aminoschutzgruppe bedeutet, kondensiert oder

d) eine Verbindung der Formel VIII

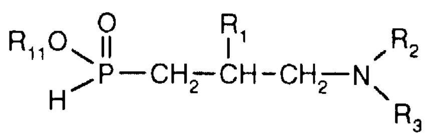

(VIII),

worin $R_{11}$ Wasserstoff oder eine Gruppe $R_4$ darstellt, mit einem Silylierungsmittel umsetzt und die erhaltene silylaktivierte Verbindung der Formel IX

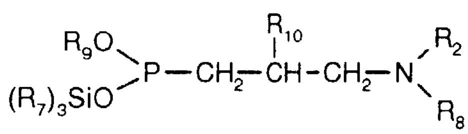

(IX),

in der $R_8$ für eine von Wasserstoff verschiedene Gruppe $R_5$ oder eine Gruppe der Formel -$Si(R_7)_3$ steht, $R_9$ eine Gruppe $R_4$ oder eine silylaktivierte Hydroxygruppen der Formel -$OSi(R_7)_3$ bedeutet und $R_{10}$ Wasserstoff oder eine Gruppe der Formel -$OSi(R_7)_3$ darstellt, wobei die Reste $R_7$ gleiche oder verschiedene aliphatische Kohlenwasserstoffreste, beispielsweise Niederalkyl, insbesondere Methyl und/oder Tertiärbutyl, bedeuten, mit einem reaktionsfähigen Ester eines aliphatischen, cycloaliphatischen, cyloaliphatisch-aliphatischen oder araliphatischen Alkohols, mit einem in $\alpha,\beta$-Stellung eine gegebenenfalls zusätzlich Doppelbindung aufweisenden aliphatischen, cycloaliphatischen, cyloal iphatisch-ali phatischen, arali phatischen oder hete roarylaliphatischen Kohlenwasserstoff, mit einem aliphatischen, cycloaliphatischen, cyloaliphatisch-aliphatischen, araliphatischen oder heteroarylaliphatischen Aldehyd oder Keton oder mit einem aliphatischen Epoxid umsetzt oder

e) zur Herstellung einer Verbindung der Formel II, in der $R_1$ Hydroxy bedeutet, eine Verbindung der Formel X

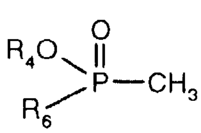

(X)

in der Form eines Metallsalzes der Formel XI

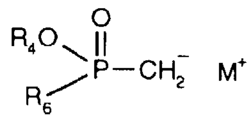

(XI),

wonn $R_6$ einen an einer gegebenenfalls vorhandenen Hydroxygruppe durch eine Gruppe $-Si(R_7)_3$ geschützten Rest bedeutet, in dem die Reste $R_7$ gleiche oder verschiedene aliphatische Kohlenwasserstoffreste, beispielsweise Niederalkyl, insbesondere Methyl und/oder Tertiärbutyl, bedeuten, und $M^+$ ein Alkali-, Erdalkali- oder Übergangsmetall-Kation darstellt, mit einem Aldehyd der Formel XII

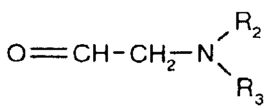

(XII)

umsetzt.

Gewünschtenfalls kann man jeweils in eine primär erhaltene Verbindung der Formel II, worin $R_3$ Wasserstoff darstellt, einen von Wasserstoff verschiedenen Rest $R_3$ einführen.

Die Einführung des Restes der Formel $R_2$ gemäss der Verfahrensvariante a) erfolgt in üblicher Weise, beispielsweise durch Umsetzung mit einer Verbindung der Formel $X-R_2$(IIIa), worin X eine reaktionsfähige veresterte Hydroxygruppen bedeutet, insbesondere in Gegenwart eines basischen Kondensationsmittels, wie einer tertiären organischen Base, beispielsweise eines Triniederalkylamin, z.B. von Triethylamin, Triisopropylamin oder Tertiärbutyl(dimethyl)amin, oder von Pyridyl, oder einer quaternären organischen Ammoniumbase, z.B. von Benzyl(trimethyl)ammoniumhydroxid. Als reaktionsfähige veresterte Hydroxygruppen kommen dabei vorzugsweise mit einer Mineralsäure veresterte Hydroxygruppen, wie Halogen, insbesondere Brom, Chlor oder Jod, oder Gruppen der Formel $R_2-O-SO_2-O-$ in Betracht.

Die Einführung des Restes der Formel $R_2$ kann aber auch durch Umsetzung mit einer Verbindung der Formel $O=R_2''$ (IIIb), worin $R_2''$ für einen zweiwertigen araliphatischen oder heteroarylaliphatischen Rest steht, dessen freie Valenzen vom gleichen C-Atom ausgehen, unter reduktiven Bedingungen, insbesondere in Gegenwart eines Alkalimetallborhydrides, z.B. von Natriumcyanoborhydrid vorzugsweise in einem Niederalkanol, wie Ethanol, Methanol oder Butanol, erfolgen.

Die Kondensation von Verbindung der Formel IV mit Aminen der Formel V gemäss der Verfahrensvariante b) erfolgt in analoger Weise wie vorstehend für die Umsetzung von Verbindungen der Formeln III und IIIa beschrieben.

In Verbindungen der Formel VII gemäss der Verfahrensvariante c) bedeutet reaktionsfähiges verestertes Hydroxy vorzugsweise Halogen, wie Brom, Jod oder Chlor, oder Sulfonyloxy, wie Niederalkansulfonyloxy, z.B. Methansulfonyloxy, oder gegebenenfalls substituiertes Benzolsulfonyloxy, z.B. Benzol-, p-Toluol- oder p-Brombenzolsulfonyloxy. Aminoschutzgruppen sind insbesondere Silylgruppen, beispielsweise der Formel $-Si(R_7)_3$, wie Triniederalkylsilyl, z.B. Trimethylsilyl. Die Umsetzung von Verbindungen der Formeln VI und reaktionsfähigen Estern VII kann in an sich bekannter Weise erfolgen, vorzugsweise unter den Bedingungen der Arbusow-Reaktion, vorteilhaft im Temperaturbereich von etwa 60° C bis etwa 180° C, z.B. bei etwa 120° C bis etwa 160° C. Bei der Umsetzung von Verbindungen der Formel VI mit Epoxiden (VII; $X_1+X_2=$ Epoxy) arbeitet man hingegen vorzugsweise in Gegenwart einer milden Lewissäure, insbesondere von Zinkchlorid, vorteilhaft in einem aprotischen Lösungsmittel.

Gemäss der Verfahrensvariante d) verwendbare Silylierungsmittel sind insbesondere Triniederalkylhalogensilane der Formel $(R_7)_3Si-Hal$ (VIIIa), worin $R_7$ Niederalkyl und Hal Halogen, wie Chlor, Brom oder Jod, bedeutet, wie Trimethylchlorsilan oder Trimethylbromsilan, oder Hexaniederalkyldisilazane der Formel $(R_7)_3Si-NH-Si(R_7)_3$ (VIIIb), worin $R_7$ Niederalkyl bedeutet, wie Hexamethyldisilazan. Das silylaktivierte Zwischenprodukte ist vorzugsweise eine Verbindung der Formel IXa

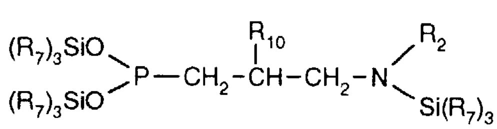

(IXa).

Die Umsetzung des Zwischenproduktes der Formel IX beziehungsweise IXa mit der den Rest R einführenden Komponente erfolgt vorzugsweise in Gegenwart eines basischen Kondensationsmittels, wie einer tertiären organischen Base, beispielsweise eines Triniederalkylamin, z.B. von Triethylamin, Triisopropylamin oder Tertiärbutyl(dimethyl)amin, oder von Pyridin, oder einer quatemären organischen Ammoniumbase, z.B. von Benzyl(trimethyl)ammoniumhydroxid.

In Ausgangsstoffen der Formel X für die Verfahrensvariante e) sind Übergangsmetall-Kationen beispielsweise Lithium-, Natrium- oder Kalium-Kationen oder Gruppen der Formel -Mg-Hal oder -Zn-Hal, wobei Hal für Chlor, Brom oder Jod steht. Die Kondensation von Verbindungen der Formeln XI und XII erfolgt in der für derartige metallorganischen Reaktionen üblichen Weise.

Die Einführung eines von Wasserstoff verschiedenen Restes $R_3$ erfolgt in üblicher Weise, insbesondere wie unter der Verfahrensvariante a) angegebenen Weise.

In Ausgangsstoffen, beispielsweise der Formeln II, V, VII, VIII, IX bzw. XII, kann man veresterte Carboxygruppen bzw. Cyanogruppen als Bestandteil des Restes $R_2$ in eines der eingangs genannten Ringsysteme einbauen. So kann man direkt oder über einen Spacer gebundenes verestertes Carboxy durch Umsetzung mit einer entsprechenden Hydroxamsäure der Formel $R_aC(=NOH)-NH_2$, worin $R_a$ beispielsweise Niederalkyl oder gegebenenfalls niederalkyliertes Amino ist, beispielsweise in Gegenwart von Natrium und pulverisierten Molekularsieben in einem Niederalkanol, wie Ethanol, in die entsprechende Verbindung der Formel I mit einer direkt oder über einen Spacer gebundenen 3-$R_a$-1,2,4-Oxadiazol-5-ylgruppe umwandeln. In analoger Weise kann man Cyano durch Behandlung mit Hydroxylaminhydrochlorid in Gegenwart von Kaliumcarbonat/Ethanol in eine Hydroxamsäuregruppe überführen, die durch Kondensation mit einem Anhydrid der Formel $(R_aCO)_2O$, wie Acetanhydrid, in die entsprechende 3-$R_a$-1,2,4-Oxadiazol-5-ylgruppe, wie 3-Methyl-1,2,4-oxadiazol-5-ylgruppe, umgewandelt werden kann. Analog kann man Cyano durch Umsetzung mit einer Hydroxamsäure der Formel $R_aC(=NOH)-NH_2$, worin $R_a$ beispielsweise Niederalkyl oder gegebenenfalls niederalkyliertes Amino ist, in 3-$R_a$-1,2,4-Triazol-5-yl überführen.

Allgemein kann man ausgehend von Verbindungen der Formel I, worin $R_2$ eine direkt oder über einen Spacer gebundene Carboxy- oder Cyanogruppe aufweist, die Carboxy- oder Cyanogruppe durch übliche Ringbildunggsmethoden in unsubstituiertes oder durch Niederalkyl, Niederalkoxy, Niederalkoxycarbonyl, Cyano, Hydroxy, Amino oder Halogen substituiertes Oxazolyl, z.B. 4-$R_a$-Oxazol-2-yl, Isoxazolyl, z.B. 3-$R_a$-Isoxazol-5-yl, Oxadiazolyl, z.B. 5-$R_a$-1,2,4-Oxadiazol-3-yl oder 5-$R_a$-1,3,4-Oxadiazol-3-yl, Triazolyl, z.B. 5-$R_a$-1,2,4-Triazol-3-yl oder Tetrazolyl, z.B.Tetrazol-5-yl überführen, wobei $R_a$ Wasserstoff oder in zweiter Linie Niederalkyl, Niederalkoxy, Niederalkoxycarbonyl, Hydroxy, Amino oder Halogen bedeutet. So kann man beispielsweise verestertes Carboxy durch Umsetzung mit einem Oxim der Formel $R_a-C(CH_3)=N-OH$ in Tetrahydrofuran und in Gegenwart von Schwefelsäure in eine 5-$R_a$-Oxazo-2-ylgruppe überführen.

In Ausgangstoffen, beispielsweise der Formeln II, V, VII, VIII, IX bzw. XII, erhaltenen Verbindungen, in denen $R_2$ eine direkt oder über einen Spacer gebundene 3-Amino-1,2,4-oxadiazol-5-ylgruppe aufweist, kann man die 3-Aminogruppe derselben in üblicher Weise, beispielsweise durch Behandeln mit Natriumnitrit in Gegenwart einer Halogenwasserstoffsaüre durch Halogen, oder durch Behandeln mit Amylnitrit durch Cyano ersetzen. In analoger Weise kann Halogen durch verethertes Hydroxy, z.B. durch Umsetzung mit einen Alkalimetallniederalkanolat durch Niederalkoxy ersetzt werden.

Erhaltene Verbindungen der Formel I können in an sich bekannter Weise in andere Verbindungen der Formel I überführt werden.

So kann man beispielsweise in Verbindungen der Formel I, worin $R_3$ Wasserstoff ist, einen von Wasserstoff verschiedenen Rest $R_3$ einführen, wobei man beispielsweise in analoger Weise wie unter der Verfahrensvariante a) beschrieben vorgeht.

Femer kann man in Verbindungen der Formel I freie und funktionell abgewandelte Carboxygruppen in üblicher Weise ineinander überführen. Beispielsweise kann man veresterte oder amidierte oder in ein heteroaromatisches Ringsystem eingebaute Carboxygruppen sowie in ein heteroaromatisches Ringsystem eingebaute Cyanogruppen in Gegenwart eines sauren oder insbesondere basischen Mittels, wie eines Alkalimetallhydroxides, von Lithiumhydroxid, zu Carboxy hydrolysieren.

In erhaltenen Verbindungen, in denen $R_2$ eine direkt oder über einen Spacer gebundene 3-Amino-1,2,4-oxadiazol-5-ylgruppe aufweist, kann man die 3-Aminogruppe derselben in üblicher Weise, beispielsweise durch Behandeln mit Natriumnitrit in Gegenwart einer Halogenwasserstoffsaüre durch Halogen, oder durch Behandeln mit Amylnitrit durch Cyano ersetzen. In analoger Weise kann Halogen durch verethertes Hydroxy, z.B. durch Umsetzung mit einen Alkalimetallniederalkanolat durch Niederalkoxy ersetzt werden.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z. B. mit Chlorwasserstoff, oder einer anderen eingangs genannten salzbildenden Säure.

Erhaltene Salze können in an sich bekannter Weise in andere Salze überführt werden, Säureadditionssalze z.B.

durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Erhaltene Racemate lassen sich femer nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z. B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl) ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-o-toluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/ oder Polyethylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Die Erfindung betrifft ebenfalls die Verwendung der Verbindungen der Formel I, vorzugsweise in Form von pharmazeutischen Präparaten.

Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 1 und etwa 50 mg/kg, insbesondere 5 und etwa 25 mg/kg und für Warmblüter mit einem Körpergewicht von etwa 70 kg vorzugsweise zwischen etwa 70 mg und etwa 3500 mg, insbesondere etwa 350 bis etwa 1750 mg, die zweckmässigerweise auf 2 bis 6, z.B. 3 oder 4 Einzeldosen aufgeteilt wird.

Die nachfolgenden Beispiele dienen zur Illustration der Erfindung; Temperaturen sind in Celsiusgraden, Drucke

in mbar angegeben.

Beispiel 1: Eine Lösung von 0.62 g 3-{N-[1-(3-Cyanophenyl)ethyl]amino}-2(S)-hyd roxy-propyl(cyclohexylmethyl) phosphinsäu reethylester in 10 ml absolutem Dichlormethan wird mit 0.97 g Trimethylbromsilan versetzt. Die entstehende Lösung wird 24 Stunden bei Raumtemperatur gerührt, die flüchtigen Anteile werden unter vermindertem Druck abgezogen und der Rückstand wird in 99.9 %-igem Methanol gelöst. Man lässt 1 Stunde bei Raumtemperatur rühren, zieht das Lösungsmittel ab und kristallisiert den Rückstand aus Ethanol. Man erhält 3-{N-[1-(3-Cyanophenyl)ethyl] amino}-2(S)-hydroxy-propyl(cyclohexylmethyl)phosphinsäure-hydrobromid vom Smp. 209-210°.

Das Ausgangsmaterial kann z.B. folgendermassen hergestellt werden:

21 g 99 %-iges Natriumhydrid wird bei Raumtemperatur unter Argon in 1000 ml wasserfreiem Tetrahydrofuran suspendiert und innerhalb von 2.5 Stunden tropfenweise mit einer Lösung von 172.2 g 1,1-Diethoxyethylphosphinsäu reethylester versetzt, wobei die Temperatur zwischen 20° und 25° gehalten wird. Die Umsetzung ist exotherm und mit Gasentwicklung verbunden. Man lässt 1.5 Stunden bei Raumtemperatur nachrühren, versetzt mit 142.2 g Brommethylcyclohexan und erhitzt 24 Stunden zum Rückfluss. Die Suspension wird auf 4° gekühlt und vorsichtig mit 250 ml Wasser versetzt. Es bilden sich zwei Phasen. Die Tetrahydrofuranphase wird abgetrennt und die Wasserphase zweimal mit je 250 ml Dichlormethan ausgezogen. Nach Eindampfen erhält man ein braunes Öl, das nach Destillation unter vermindertem Druck 1,1-Diethoxyethyl(cyclohexylmethyl)phosphinsäureethylester vom Kp = 95° ($1.8x10^{-4}$ mbar) ergibt.

Eine Lösung von 213 g 1,1-Diethoxyethyl(cyclohexylmethyl)phosphinsäureethylester in 600 ml eines 600 ml eines Gemisches von Dichlormethan und Ethanol (90:10 Vol-%) wird mit 150 g Trimethylchlorsilan versetzt und 2 bis 3 Tage bei Raumtemperatur gerührt. Das Lösungsmittel wird abgezogen und der Rückstand unter vermindertem Druck destilliert. Man erhält P-(Cyclohexylmethyl)phosphinsäureethylester vom Kp = 50° ($2x10^{-4}$ mbar).

Eine Lösung von 51.0 g P-(Cyclohexylmethyl)phosphinsäureethylester in 750 ml absolutem Tetrahydrofuran und 29.8 g Triethylamin wird mit 32 g Trimethylchlorsilan versetzt. Es bildet sich ein weisser Niederschlag. Die erhaltene Suspension wird 24 Stunden bei Raumtemperatur gerührt, unter Argon filtriert und eingedampft. Das zurückbleibende farblose Öl wird mit 24 g (R)-Epichlorhydrin und 6.6 g wasserfreiem Zinkchlorid versetzt. Die Reaktion ist stark exotherm. Nach Abklingen derselben wird das Reaktionsgemisch 24 Stunden auf 60° erwärmt, dann auf Raumtemperatur abgekühlt, mit 250 ml Dichlormethan versetzt und mit Wasser gewaschen. Die organische Phase wird abgetrennt, über Natriumsulfat getrocknet und eingedampft. Der Rückstand wird in einem Gemisch von 99 ml Methanol und 1 ml Essigsäure aufgenommen, 24 Stunden bei Raumtemperatur gerührt und eingedampft. Der Rückstand wird an Silicagel chromatographiert und ergibt 3-Chlor-2(R)-hydroxy-propyl(cyclohexylmethyl)-phosphinsäureethylester als farbloses Öl.

Eine Mischung von 5.65 g 3-Chlor-2(R)-hydroxy-propyl(cyclohexylmethyl)--phosphinsäureethylester, 2.92 g N-[1-(3-Cyanophenyl)ethyl]amin und 2.60 g Hünig'scher Base und 10 ml Ethanol wird 5 Tage zum Rückfluss erhitzt, dann auf Raumtemperatur abgekühlt und eingedampft. Chromatographie des Rückstandes an Silicagel ergibt 3-{N-[1-(3-Cyanophenyl)ethyl]amino}-2(S)-hydroxypropyl(cyclohexylmethyl)phosphinsäureethylester als Öl; $[\alpha]_D^{20}$ = +8.6 (c = 1.225 in Trichlormethan).

Beispiel 2: Eine Lösung von 3.0 g 3-{N-[1-(3-Cyanophenyl)ethyl]amino}-2(S)-hydroxy-propyl(cyclohexylmethyl) phosphinsäureethylester in 10 ml Ethanol wird mit einer Lösung von 0.4 g Lithiumhydroxid in 10 ml Wasser versetzt und 24 Stunden zum Rückfluss erhitzt. Man kühlt auf 4° ab und neutralisiert mit wässriger Phosphorsäure. Das Lösungsmittel wird abgezogen und der Rückstand in warmem Methanol aufgenommen und filtriert. Nach Abziehen des Lösungsmittel und Kristallisation aus Ethanol/Aceton erhält man Lithium-3-{N-[1-(3-Carboxyphenyl)ethyl]amino}-2(S)-hydroxy-propyl(cyclohexylmethyl)phosphinat vom Smp. 160-184°.

Beispiel 3: In analoger Weise wie in Beispiel 1 beschrieben erhält man durch Umsetzung von 3-Chlor-2(R)-hydroxy-propyl(cyclohexylmethyl)-phosphinsäureethylester mit 1-(3-Methoxycarbonylphenyl)ethylamin Lithium-3-{N[1-(3-Carboxyphenyl)ethyl]amino}-2(S)-hydroxy-propyl-(cyclohexylmethyl)phosphinat vom Smp. 160-184°, das mit dem Produkt gemäss Beispiel 2 identisch ist.

Beispiel 4: In analoger Weise wie in Beispiel 1 beschrieben erhält man

a) 3-{N-[1-(4-Cyanophenyl)ethyl]amino}-2(S)-hydroxy-propyl-(cyclohexylmethyl)phosphinsäure vom Smp. 204-209°;

b) 3-{N-[1(S)-(4-Cyanophenyl)ethyl]amino}-2(S)-hydroxy-propyl-(cyclohexyl methyl)phosphinsäure-hydrobromid vom Smp. 217-218°;

c) 3-{N-[1(R)-(4-Cyanophenyl)ethyl]amino}-2(S)-hydroxy-propyl-(cyclohexylmethyl)phosphinsäure-hydrobromid vom Smp. 210-212°;

d) 3-{N-[1(S)-(3-Cyanophenyl)ethyl]amino}-2(S)-hydroxy-propyl-(cyclohexylmethyl)phosphinsäure-hydrobromid vom Smp. 224-225°;

e) 3-{N-[1(S)-(3-Cyanophenyl)ethyl]amino}-2(R)-hydroxy-propyl-(cyclohexylmethyl)phosphinsäu re-hydrobrom id

vom Smp. 168-170°;

f) 3-{N-[1(R)-(3-Cyanophenyl)ethyl]amino}-2(S)-hydroxy-propyl-(cyclohexylmethyl)phosphinsäure-hydrobromid vom Smp. 220-222°.

Beispiel 5: In analoger Weise wie in Beispiel 2 beschrieben erhält man

a) 3-{N-[1-(4-Carboxyphenyl)ethyl]amino}-2(S)-hydroxy-propyl-(cyclohexylmethyl)phosphinsäure vom Smp. 180-187°;

b) Lithium-3-{N-[1(S)-(4-Carboxyphenyl)ethyl]amino}-2(S)-hydroxy-propyl-(cyclohexylmethyl)phosphinat vom Smp. 184-186° ;

c) Lithium-3-{N-[1(R)-(4-Carboxyphenyl)ethyl]amino}-2(S)-hydroxy-propyl-(cyclohexylmethyl)phosphinat vom Smp. 190-192° ;

d) Lithium-3-{N-[1(S)-(3-Carboxyphenyl)ethyl]amino}-2(S)-hydroxy-propyl-(cyclohexylmethyl)phosphinat vom Smp. 180-182° ;

e) Lithium-3-{N-[1(S)-(3-Carboxyphenyl)ethyl]amino}-2(R)-hydroxy-propyl-(cyclohexylmethyl)phosphinat vom Smp. 184-186° ;

f) Lithium-3-{N-[1 (R)-(3-Carboxyphenyl)ethyl]amino}-2(S)-hydroxy-propyl-(cyclohexylmethyl)phosphinat vom Smp. 181-183° ;

g) Lithium-3-{N-[1(R)-(3-Carboxyphenyl)ethyl]amino}-2(S)-hydroxy-propyl-(cyclohex-3-enylmethyl)phosphinat;

h) Lithium-3-{N-[1(S)-(4-Carboxyphenyl)ethyl]amino}-2(S)-hydroxy-propyl-(cyclohex-3-enylmethyl)phosphinat.

Beispiel 6: In analoger Weise wie in Beispiel 1 beschrieben erhält man

a) 3-{N-[1-(4-Cyanophenyl)ethyl]amino}-2(S)-hydroxy-propyl(benzyl)-phosphinsäure vom Smp. 110-120°;

b) 3-{N-[1-(3-Cyanophenyl)ethyl]amino}-2(S)-hydroxy-propyl(benzyl)-phosphinsäure-hydrobromid vom Smp. 134-138°.

Beispiel 7: In analoger Weise wie in Beispiel 2 beschrieben erhält man

a) 3-{N-[1-(4-Carboxyphenyl)ethyl]amino}-2(S)-hydroxy-propyl(benzyl)-phosphinsäure vom Smp. 188-191°;

b) Lithium-3-{N-[1-(3-Carboxyphenyl)ethyl]amino}-2(S)-hydroxy-propyl(benzyl)-phosphinat vom Smp. 185-190°;

c) 3-{N-[1-(3-Carboxy-4-methoxyphenyl)ethyl]amino}-2(S)-hydroxy-propyl-(benzyl)phosphinsäure.

Beispiel 8: In analoger Weise wie in den Beispielen 1 bis 3 beschrieben kann man femer herstellen:

3-{{N-{1-[3-(Isoxazol-5-yl)phenyl]ethyl}amino}}-2(S)-hydroxy-propyl(cyclohexylmethyl)phosphinsäure;

3-{{N-(1-[3-(Isoxazol-2-yl)phenyl]ethyl}amino}}-2(S)-hydroxy-propyl(cyclohexylmethyl)phosphinsäure;

3-{{N-{1-[3-(1,2,4-Oxadiazol-5-yl)phenyl]ethyl}amino}}-2(S)-hydroxy-propyl(cyclohexylmethyl)phosphinsäure;

3-{{N-{1-[3-(1,2,4-Oxadiazol-3-yl)phenyl]ethyl}amino}}-2(S)-hydroxy-propyl(cyclohexylmethyl)phosphinsäure;

3-{{N-{1-[3-(1,2,4-Triazol-3-yl)phenyl]ethyl}amino}}-2(S)-hydroxy-propyl-(cyclohexylmethyl)phosphinsäure;

3-{{N-{1-[3-(1,2,4-Triazol-5-yl)phenyl]ethyl}amino}}-2(S)-hydroxy-propyl-(cyclohexyl methyl)phosphinsäure;

3-{{N-{1-[3-(Tetrazol-5-yl)phenyl]ethyl}amino}}-2(S)-hydroxy-propyl(cyclohexylmethyl)phosphinsäure;

3-{{N-{1-[4-(3-Amino-1,2,4-Oxadiazol-5-yl)phenyl]ethyl}amino}}-2(S)-hydroxy-propyl(cyclohexylmethyl)phosphinsäure;

3-{{N-{1-[4-(3-Amino-1,2,4-Oxadiazol-5-yl)phenyl]ethyl}amino}}-2(S)-hydroxypropyl(benzyl)phosphinsäure;

3-{N-[1-(4-Carboxymethylphenyl)ethyl]amino}-2(S)-hydroxy-propyl(cyclohexylmethyl)phosphinsäure;

3-{N-[1-(2-Carboxymethylpyrid-4-yl)ethyl]amino}-2(S)-hydroxy-propyl(cyclohexylmethyl)phosphinsäure;

3-{N-[1-(4-Carboxymethylphenyl)ethyl]amino}-2(S)-hydroxy-propyl(cyclohexylmethyl)phosphinsäure und

3-{N-[1-(4-Carboxymethylphenyl)ethyl]amino}-2(S)-hydroxy-propyl(benzyl)phosphinsäure.

Beispiel 9: In analoger Weise wie in Beispiel 2 beschrieben erhält man

a) Lithium-3-{N-[1-(R)-(3-Carboxyphenyl)ethyl]amino}-2-(S)-hyroxy-propyl(diethoxymethyl)phosphinat, Smp. 166-168°;
b) Lithium-3-{N-[1-(S)-(3-Carboxyphenyl)ethyl]amino}-2-(S)-hyroxy-propyl(diethoxymethyl)phosphinat, Smp. 168-170°.

Beispiel 10: Eine Lösung von 0.25 g 3-{{N-{1-[4-(3-Amino-1,2,4-oxadiazol-5-yl)phenyl]ethyl}amino}}-2(S)-hydroxypropyl(cyclohexylmethyl)phosphinsäureethylester in 1 ml Ethanol wird mit einer Lösung von 27 mg Lithiumhydroxid versetzt und 24 Stunden zum Rückfluss erhitzt. Dann lässt man auf Raumtemperatur abkühlen und stellt mit konzentrierter Phosphorsäure auf pH 7. Es bildet sich eine weisser Niederschlag. Die Suspension desselben wird zur Trockne eingedampft, der Rückstand in Methanol aufgenommen und abfiltriert. Das klare Filtrat wird 24 Stunden bei 4° stehengelassen. Es scheiden sich Kristalle ab, die abfiltriert und unter vermindertem Druck getrocknet werden. Man erhält 3-{{N-{1-[4-(3-Amino-1,2,4-oxadiazol-5-yl)phenyl]ethyl}amino}}-2(S)-hydroxypropyl(cyclohexylmethyl)phosphinsäure in Form gelber Kristalle vom Smp. 222.5-224°; Massenspektrum: $M^{+}$-1= 422; $^{1}$H-NMR-Spektrum ($CD_3OD$), δ(ppm)= 8.14 (2H,m), 7.68 (2H,m), 4.50(1H,m), 4.20 (1H,m), 3.19 (1H,dd), 3.02 (1H,dd), 2.10-1.54 (12H,m), 1.39-0.95 (6H,m).

Das Ausgangsmaterial kann z.B. folgendermassen hergestellt werden:

10 g 4-Acetylbenzoesäureethylester und 40 g Ammoniumacetat werden in 210 ml absolutem Methanol gelöst und mit 3.63 g Natriumcyanoborhydrid versetzt. Die Reaktionslösung wird 24 Stunden bei Raumtemperatur gerührt, auf 4° gekühlt und durch Zugabe von konzentrierter Salzsäure auf pH 1 gestellt. Das Methanol wird unter vermindertem Druck abgezogen und die zurückbleibende Suspension filtriert. Das wässrige Filtrat wird mit Diethylether gewaschen, bei 4° durch Zugabe von festem Natriumhydroxid auf pH 10 gestellt und mit Diethylether ausgezogen. Man trocknet über Natriumsulfat, dampft unter vermindertem Druck zur Trockne ein und destilliert das zurückbleibende Öl unter vermindertem Druck. Man erhält 1-(4-Carboxyphenyl)ethylamin, Kp= 130-140° ($6x10^{-2}$ mbar).

Man löst 0.8 g Natrium in 60 ml absolutem Ethanol und fügt 12 g Molekularsieb (4Å) sowie 2.63 g N-Hydroxyguanidin hinzu, lässt Stunde bei Raumtemperatur rühren und gibt dann 1.14 g 1-(4-Carboxyphenyl)ethylamin zu. Die erhaltene gelblich-trübe Lösung wird 2 Stunden zum Rückfluss erhitzt, nach welcher Zeit mittels Dünnschichtchromatographie vollständige Umsetzung festgestellt werden kann. Man filtriert und zieht das Lösungsmittel unter vermindertem Druck ab. Man digeriert den Rückstand mit Wasser, kühlt im Eisbad, filtriert den Niederschlag ab, wäscht mit wenig Wasser und trocknet unter vermindertem Druck. Man erhält 1-[4-(3-Amino-1,2,4-oxadiazol-5-yl)phenyl]ethylamin vom Smp. 150-151°.

Eine Lösung von 0.8 g 1-[4-(3-Amino-1,2,4-oxadiazol-5-yl)phenyl]ethylamin und 1.106 g 3-Chlor-2(R)-hydroxypropyl(cyclohexylmethyl)phosphinsäureethylester in 5 ml Ethanol, enthaltend 0.5 g Hünig'scher Base wird 24 Stunden zum Rückfluss erhitzt. Man kühlt auf Raumtemperatur und zieht das Lösungsmittel unter vermindertem Druck ab. Chromatographie des Eindampfrückstandes an Silicagel ergibt 3-{{N-{1-[4-(3-Amino-1,2,4-oxadiazol-5-yl)phenyl] ethyl}amino}}-2(S)-hydroxy-propyl(cyclohexylmethyl)phosphinsäureethylester als fahlgelben Festkörper; Massenspektrum: $M^{+}$-1= 449; $^{1}$H-NMR-Spektrum ($CD_3OD$), δ(ppm)= 8.00 (2H,m), 7.45 (2H,m), 4.21-3.92 (3H,m), 3.80 (1H, m), 2.70-2.30 (2H,m), 2.00-1.55 (9H,m), 1.40-0.90 (8H,m).

Beispiel 11: Eine Lösung von 0.125 g Lithium-3-{N-[1(R)-(3-Carboxyphenyl)ethyl]amino}-2(S)-hydroxy-propyl(cyclohex-3-enylmethyl)phosphinat, in 5 ml Ethanol wird mit 20 mg 5%-iger Palladiumkohle versetzt und bei Raumtemperatur und Normaldruck 15 Minuten hydriert. Der Katalysator wird durch Celite® abfiltriert und des Filtrat mit ethanolischer Salzsäure auf pH 1 gestellt. Abziehen des Lösungsmittels und Umkristallisieren aus Isopropanol ergibt Lithium-3-{N-[1(R)-(3-Carboxyphenyl)ethyl]amino}-2(S)-hydroxy-propyl(cyclohexylmethyl)phosphinat, identisch mit der Verbindung gemäss Beispiel 5(f).

In analoger Weise kann man auch Lithium-3-{N-[1(S)-(4-Carboxyphenyl)ethyl]amino}-2(S)-hydroxy-propyl(cyclohex-3-enylmethyl)phosphinat, identisch mit der Verbindung gemäss Beispiel 5(g), herstellen.

Beispiel 12: In analoger Weise wie in Beispiel 10 beschrieben kann man ferner herstellen:

3-{{N-{1-[4-(3-Amino-1,2,4-oxadiazol-5-yl)phenyl]ethyl}amino}}-2(S)-hydroxy-propyl(benzyl)phosphinsäure;

3-{{N-{1-[3-(3-Amino-1,2,4-oxadiazol-5-yl)phenyl]ethyl}amino}}-2(S)-hydroxy-propyl(benzyl)phosphinsäure;

3-{{N-{1-[3-(3-Amino-1,2,4-oxadiazol-5-yl)phenyl]ethyl}amino}}-2(S)-hydroxy-propyl(cyclohexylmethyl)phosphinsäure;

3-{{N-{1-[3-(3-Chlor-1,2,4-oxadiazol-5-yl)phenyl]ethyl}amino}}-2(S)-hydroxy-propyl(cyclohexylmethyl)phosphinsäure;

3-{{N-{1-[3-(3-Chlor-1,2,4-oxadiazol-5-yl)phenyl]ethyl}amino}}-2(S)-hydroxy-propyl(benzyl)phosphinsäure;

3-{{N-{1-[4-(3-Chlor-1,2,4-oxadiazol-5-yl)phenyl]ethyl}amino}}-2(S)-hydroxy-propyl(benzyl)phosphinsäure;

3-{{N-{1-[4-(3-Chlor-1,2,4-oxadiazol-5-yl)phenyl]ethyl}amino}}-2(S)-hydroxy-propyl(cyclohexylmethyl)phosphinsäure;

3-{{N-{1-[4-(1,2,4-Oxadiazol-5-yl)phenyl]ethyl}amino}}-2(S)-hydroxy-propyl(cyclohexylmethyl)phosphinsäure;

3-{{N-{1-[4-(1,2,4-Oxadiazol-5-yl)phenyl]ethyl}amino}}-2(S)-hydroxy-propyl(benzyl)phosphinsäure;

3-{{N-{1-[3-(1,2,4-Oxadiazol-5-yl)phenyl]ethyl)amino}}-2(S)-hydroxy-propyl(cyclohexylmethyl)phosphinsäure;

3-{{N-{1-[3-(1,2,4-Oxadiazol-5-yl)phenyl]ethyl}amino}}-2(S)-hydroxy-propyl(benzyl)phosphinsäure;

3-{N-[1-(3-Carboxy-4-methoxyphenyl)ethyl]amino}-2(S)-hydroxy-propyl(pyrid-3-yl-methyl)phosphinsäure;

3-{N-[1-(3-Carboxy-4-methoxyphenyl)ethyl]amino}-2(S)-hydroxy-propyl(thien-2-yl-methyl)phosphinsäure;

3-{N-[1-(3-Carboxy-4-methoxyphenyl)ethyl]amino}-2(S)-hydroxy-propyl(fur-2-yl-methyl)phosphinsäure.

Beispiel 13: Eine Lösung von 1.0 g Lithium-3-{N-[1(S)-(3-Carboxyphenyl)ethyl]amino}-2(S)-hydroxy-propyl(cyclohexylmethyl)phosphinat in 2 ml Wasser wird auf eine DOWEX' 50Wx8 lonnenaustauschersäule (40-60 Mesh) aufgebracht und mit Wasser eluiert. Die Ninhydrin-positiven Fraktionen werden vereinigt und unter vermindertem Druck zur Trockne eingedampft. Durch Kristallisation des schaumigen Eindampfrückstandes aus Ethanol/Tetrahydrofuran erhält man 3-{N-[1(S)-(3-Carboxyphenyl)ethyl]amino]-2(S)-hydroxy-propyl(cyclohexylmethyl)phosphinsäure vom Smp. 225-228°.

Beispiel 14: Tabletten, enthaltend je 200 mg 3-{N-[1-(3-Cyanophenyl)ethyl]amino}-2(S)-hydroxy-propyl(cyclohexylmethyl)phosphinsäure oder ein Salz davon können wie folgt hergestellt werden:

| Zusammensetzung (10000 Tabletten) | |
|---|---|
| Wirkstoff | 2000,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 295,0 mg Gewicht und 50,0 mg Wirkstoffgehalt, die gewünschtenfalls mit Teilkerben zur feineren Anpassung der Dosierung versehen sein können.

Beispiel 15: Lacktabletten, enthaltend je 400 mg 3-{N-[1-(3-Cyanophenyl)ethyl]amino}-2(S)-hydroxy-propyl(cyclohexylmethyl)phosphinsäure oder ein Salz davon können wie folgt hergestellt werden:

| Zusammensetzung (für 1000 Lacktabletten) | |
|---|---|
| Wirkstoff | 400,0 g |
| Lactose | 100,0 g |
| Maisstärke | 70,0 g |

(fortgesetzt)

| Zusammensetzung (für 1000 Lacktabletten) | |
|---|---|
| Talk | 8,5 g |
| Calciumstearat | 1,5 g |
| Hydroxypropylmethylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 583 mg) verpresst und diese mit einer Lösung der Hydroxypropyl-methylcellulose und des Schellacks in Methylenchlorid lackiert; Endgewicht der Lacktablette: 585 mg.

Beispiel 16:

Gelatinesteckkapseln, enthaltend 500 mg Wirkstoff, z.B. 3-{N-[1-(3-Cyanophenyl)ethyl]amino}-2(S)-hydroxy-propyl(cyclohexylmethyl)phosphinsäure oder ein Salz davon, können z.B. folgendermassen hergestellt werden:

| Zusammensetzung (für 1000 Kapseln) | |
|---|---|
| Wirkstoff | 500,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird emeut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 790 mg der erhaltenen Formulierung in Gelatinesteckkapseln passender Grösse abgefüllt.

Beispiel 17: Eine 5%-ige Injektions- oder Infusionslösung von 3-{N-[1-(3-Cyanophenyl)ethyl]amino}-2(S)-hydroxy-propyl(cyclohexylmethyl)phosphinsäure oder ein Salz davon, kann beispielsweise folgendermassen hergestellt werden:

| Zusammensetzung (für 1000 bzw. 400 Ampullen) | |
|---|---|
| Wirkstoff | 125,0 g |
| Natriumchlorid | 22,5 g |
| Phosphatpuffer pH=7.4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 50 bzw. 125 mg Wirkstoff enthalten.

Beispiel 18: In analoger Weise wie in den vorstehenden Formulierungsbeispielen beschrieben kann man auch pharmazeutische Präparate, enthaltend eine andere Verbindung der Formel I gemäss einem der Beispiele 1 bis 13 herstellen.

## Patentansprüche

**1.** Eine N-Aralkyl- und N-Heteroaralkylaminoalkanphosphinsäure der Formel I

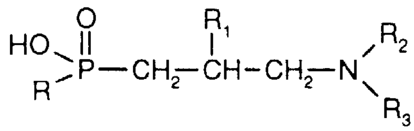

(I),

worin R $C_3$-$C_7$-Alkyl, α,α-Di-$C_1$-$C_4$-alkoxy-$C_1$-$C_4$-alkyl, $C_3$-$C_6$-Cycloalkyl-$C_1$-$C_4$-alkyl, $C_3$-$C_6$-Cycloalkenyl-$C_1$-$C_4$-alkyl, oder unsubstituiertes oder durch $C_1$-$C_4$-Alkyl, $C_1$-$C_4$-Alkoxy, Hydroxy und/oder Halogen, mono-, di- oder trisubstituiertes Phenyl-$C_1$-$C_4$-alkyl bedeutet, $R_1$ Wasserstoff oder Hydroxy darstellt, $R_2$ einen durch Carboxy, $C_1$-$C_4$-Alkoxycarbonyl, Cyano, Carbamoyl, N-Mono- oder N,N-Di-$C_1$-$C_4$-alkylcarbamoyl, Carboxy-$C_1$-$C_4$-alkyl, $C_1$-$C_4$-Alkoxycarbonyl-$C_1$-$C_4$-alkyl, Cyano-$C_1$-$C_4$-alkyl, Carbamoyl-$C_1$-$C_4$-alkyl, N-Mono- oder N,N-Di-$C_1$-$C_4$-alkylcarbamoyl-$C_1$-$C_4$-alkyl, unsubstituiertes oder durch $C_1$-$C_4$-Alkyl, $C_1$-$C_4$-Alkoxy, $C_1$-$C_4$-Alkoxycarbonyl, Cyano, Hydroxy, Amino oder Halogen substituiertes Oxazolyl, Isoxazolyl, Oxadiazolyl, Triazolyl, Tetrazolyl, Oxazolyl-$C_1$-$C_4$-alkyl, Isoxazolyl-$C_1$-$C_4$-alkyl, Oxadiazolyl-$C_1$-$C_4$-alkyl, Triazolyl-$C_1$-$C_4$-alkyl oder Tetrazolyl-$C_1$-$C_4$-alkyl ein- oder zweifach und gegebenenfalls zusätzlich durch $C_1$-$C_4$-Alkoxy, Polyfluor-$C_1$-$C_4$-alkoxy, Halogen oder Polyfluor-$C_1$-$C_4$-alkyl substituierten Phenyl-, Thienyl-, Furyl- oder Pyridyl-$C_1$-$C_4$-alkylrest und $R_3$ Wasserstoff oder $C_1$-$C_4$-Alkyl bedeutet, oder ein Salz davon.

**2.** Eine Verbindung gemäss Anspruch 1 der Formel I, worin R $C_3$-$C_5$-Alkyl, α,α-Di-$C_1$-$C_4$-alkoxymethyl, $C_3$-$C_6$-Cycloalkyl-$C_1$-$C_4$-alkyl oder Benzyl bedeutet, $R_1$ Wasserstoff oder Hydroxy darstellt, $R_2$ einen durch Carboxy, $C_1$-$C_4$-Alkoxycarbonyl, Cyano, Carbamoyl, Carboxy-$C_1$-$C_4$-alkyl, $C_1$-$C_4$-Alkoxycarbonyl-$C_1$-$C_4$-alkyl, Cyano-$C_1$-$C_4$-alkyl, Carbamoyl-$C_1$-$C_4$-alkyl, Isoxazol-5-yl, Isoxazol-2-yl, 1,2,4-Oxadiazol-5-yl, 1,2,4-Oxadiazol-3-yl, 1,3,4-Oxadiazol-3-yl, 1,2,4-Triazol-3-yl, 1,2,4-Triazol-5-yl oder Tetrazol-5-yl, Isoxazol-5-ylmethyl, Isoxazol-2-ylmethyl, 1,2,4-Oxadiazol-5-ylmethyl, 1,2,4-Oxadiazol-3-ylmethyl, 1,3,4-Oxadiazol-3-ylmethyl, 1,2,4-Triazol-3-ylmethyl, 1,2,4-Triazol-5-ylmethyl oder Tetrazol-5-ylmethyl, ein- oder zweifach und gegebenenfalls zusätzlich durch $C_1$-$C_4$-Alkoxy, Trifluormethoxy, Halogen oder Trifluormethyl substituierten α-Phenyl-$C_1$-$C_4$-alkyl- oder α-Pyridyl-$C_1$-$C_4$-alkylrest darstellt und $R_3$ Wasserstoff bedeutet, oder ein Salz davon.

**3.** Eine Verbindung gemäss Anspruch 1 der Formel I, wonn R α,α-Di-$C_1$-$C_4$-alkoxymethyl, $C_3$-$C_6$-Cycloalkyl-$C_1$-$C_4$-alkyl, $C_3$-$C_6$-Cycloalk-3-enyl-$C_1$-$C_4$-alkyl oder Benzyl bedeutet, $R_1$ Hydroxy darstellt, $R_2$ einen durch Carboxy, Cyano oder unsubstituiertes oder durch Amino oder Halogen substituiertes 1,2,4-Oxadiazol-5-yl substituierten Phenyl-$C_1$-$C_4$-alkylrest darstellt und $R_3$ Wasserstoff bedeutet, oder ein Salz davon.

**4.** Eine Verbindung gemäss Anspruch 1 der Formel I, worin R $C_3$-$C_5$-Alkyl, α,α-Di-$C_1$-$C_4$-alkoxymethyl, $C_3$-$C_6$-Cycloalkyl-$C_1$-$C_4$-alkyl oder Benzyl bedeutet, $R_1$ Wasserstoff oder Hydroxy darstellt, $R_2$ einen durch Carboxy, $C_1$-$C_4$-Alkoxycarbonyl, Cyano oder Carbamoyl ein- oder zweifach und gegebenenfalls zusätzlich durch $C_1$-$C_4$-Alkoxy, Trifluormethoxy, Halogen oder Trifluormethyl substituierten Phenyl-$C_1$-$C_4$-alkylrest darstellt und $R_3$ Wasserstoff bedeutet, oder ein Salz davon.

**5.** Eine Verbindung gemäss einem der Ansprüche 1 bis 5 der Formel I, in der das mit der Gruppe $R_1$ verbundene C-Atom der Propylenkette, sofern $R_1$ Hydroxy ist, und/oder ein chirales α-C-Atom des aliphatischen Teils des Restes $R_2$, sofern ein solches vorhanden ist, jeweils (S)-Konfiguration aufweist.

**6.** 3-{N-[1-(3-Cyanophenyl)ethyl]amino}-2(S)-hydroxy-propyl(cyclohexylmethyl)phosphinsäure oder ein Salz davon.

**7.** 3-{N-[1-(3-Carboxyphenyl)ethyl]amino}-2(S)-hydroxy-propyl(cyclohexylmethyl)phosphinsäure oder ein Salz davon.

**8.** 3-{N-[1-(4-Cyanophenyl)ethyl]amino}-2(S)-hydroxy-propyl(cyclohexylmethyl)phosphinsäure oder ein Salz davon.

**9.** 3-{N-[1(S)-(4-Cyanophenyl)ethyl]amino}-2(S)-hydroxy-propyl(cyclohexylmethyl)phosphinsäure oder ein Salz davon.

**10.** 3-{N-[1(R)-(4-Cyanophenyl)ethyl]amino}-2(S)-hydroxy-propyl(cyclohexylmethyl)phosphinsäure oder ein Salz davon.

**11.** 3-{N-[1(S)-(3-Cyanophenyl)ethyl]amino}-2(S)-hydroxy-propyl(cyclohexylmethyl)phosphinsäure oder ein Salz davon.

**12.** 3-{N-[1(S)-(3-Cyanophenyl)ethyl]amino}-2(R)-hydroxy-propyl-(cyclohexylmethyl)phosphinsäure oder ein Salz davon.

**13.** 3-{N-[1(R)-(3-Cyanophenyl)ethyl]amino}-2(S)-hydroxy-propyl-(cyclohexylmethyl)phosphinsäure oder ein Salz davon.

**14.** 3-{N-[1-(4-Carboxyphenyl)ethyl]amino}-2(S)-hydroxy-propyl-(cyclohexylmethyl)phosphinsäure oder ein Salz davon.

**15.** 3-{N-[1(S)-(4-Carboxyphenyl)ethyl]amino}-2(S)-hydroxy-propyl-(cyclohexylmethyl)phosphinsäure oder ein Salz davon.

**16.** 3-{N-[1(R)-(4-Carboxyphenyl)ethyl]amino}-2(S)-hydroxy-propyl-(cyclohexylmethyl)phosphinsäure oder ein Salz davon.

**17.** 3-{N-[1(S)-(3-Carboxyphenyl)ethyl]amino}-2(S)-hydroxy-propyl-(cyclohexylmethyl)phosphinsäure oder ein Salz davon.

**18.** 3-{N-[1(S)-(3-Carboxyphenyl)ethyl]amino}-2(R)-hydroxy-propyl-(cyclohexylmethyl)phosphinsäure oder ein Salz davon.

**19.** 3-{N-[1(R)-(3-Carboxyphenyl)ethyl]amino}-2(S)-hydroxy-propyl-(cyclohexylmethyl)phosphinsäure oder ein Salz davon.

**20.** 3-{N-[1(R)-(3-Carboxyphenyl)ethyl]amino}-2(S)-hydroxy-propyl(cyclohex-3-enylmethyl)phosphinsäure oder ein Salz davon.

**21.** 3-{N-[1(S)-(4-Carboxyphenyl)ethyl]amino}-2(S)-hydroxy-propyl(cyclohex-3-enylmethyl)phosphinsäure oder ein Salz davon.

**22.** 3-{N-[1-(4-Cyanophenyl)ethyl]amino}-2(S)-hydroxy-propyl(benzyl)phosphinsäure oder ein Salz davon.

**23.** 3-{N-[1-(3-Cyanophenyl)ethyl]amino}-2(S).hydroxy-propyl(benzyl)phosphinsäure oder ein Salz davon.

**24.** 3-{N-[1-(4-Carboxyphenyl)ethyl]amino}-2(S)-hydroxy-propyl(benzyl)phosphinsäure oder ein Salz davon.

**25.** 3-{N-[1-(4-Carboxyphenyl)ethyl]amino}-2(S)-hydroxy-propyl(benzyl)phosphinsäure oder ein Salz davon.

**26.** 3-{N-[1-(3-Carboxyphenyl)ethyl]amino}-2(S)-hydroxy-propyl(benzyl)phosphinsäure oder ein Salz davon.

**27.** 3-{N-[1-(3-Carboxy-4-methoxyphenyl)ethyl]amino}-2(S)-hydroxy-propyl(benzyl)phosphinsäure oder ein Salz davon.

**28.** 3-{{N-{1-[3-(Isoxazol-5-yl)phenyl]ethyl}amino}}-2(S)-hydroxy-propyl(cyclohexylmethyl)phosphinsäure;

3-{{N-{1-[3-(Isoxazol-2-yl)phenyl]ethyl}amino}}-2(S)-hydroxy-propyl(cyclohexylmethyl)phosphinsäure;

3-{{N-{1-[3-(1,2,4-Oxadiazol-5-yl)phenyl]ethyl}amino}}-2(S)-hydroxy-propyl(cyclohexylmethyl)phosphinsäure;

3-{{N-{1-[3-(1,2,4-Oxadiazol-3-yl)phenyl]ethyl}amino}}-2(S)-hydroxy-propyl(cyclohexylmethyl)phosphinsäure;

3-{{N-{1-[3-(1,2,4-Triazol-3-yl)phenyl]ethyl}amino}}-2(S)-hydroxy-propyl(cyclohexylmethyl)phosphinsäure;

3-{{N-{1-[3-(1,2,4-Triazol-5-yl)phenyl]ethyl}amino}}-2(S)-hydroxy-propyl(cyclohexylmethyl)phosphinsäure;

3-{{N-{1-[3-(Tetrazol-5-yl)phenyl]ethyl}amino}}-2(S)-hydroxy-propyl(cyclohexylmethyl)phosphinsäure;

3-{{N-{1-[4-(3-Amino-1,2,4-Oxadiazol-5-yl)phenyl]ethyl}amino}}-2(S)-hydroxy-propyl(cyclohexylmethyl)phosphinsäure;

3-{{N-{1-[4-(3-Amino-1,2,4-Oxadiazol-5-yl)phenyl]ethyl}amino}}-2(S)-hydroxy-propyl(benzyl)phosphinsäure;

3-{N-[1-(4-Carboxymethylphenyl)ethyl]amino}-2(S)-hydroxy-propyl(cyclohexylmethyl)phosphinsäure;

3-{N-[1-(2-Carboxymethylpyrid-4-yl)ethyl]amino}-2(S)-hydroxy-propyl(cyclohexylmethyl)phosphinsäure;

3-{N-[1-(4-Carboxymethylphenyl)ethyl]amino}-2(S)-hydroxy-propyl(cyclohexylmethyl)phosphinsäure oder

3-{N-[1-(4-Carboxymethylphenyl)ethyl]amino}-2(S)-hydroxy-propyl(benzyl)phosphinsäure oder jeweils ein Salz davon.

**29.** 3-{N-[1-(R)-(3-Carboxyphenyl)ethyl]amino}-2-(S)-hyroxy-propyl(diethoxymethyl)phosphinsäure;

3-{N-[1-(S)-(3-Carboxyphenyl)ethyl]amino}-2-(S)-hyroxy-propyl(diethoxymethyl)phosphinsäure;

3-{{N-{1-[4-(3-Amino-1,2,4-oxadiazol-5-yl)phenyl}amino}}-2(S)-hydroxy-propyl(cyclohexylmethyl)phosphinsäure;

3-{{N-{1-[4-(3-Amino-1,2,4-oxadiazol-5-yl)phenyl}amino}}-2(S)-hydroxy-propyl(benzyl)phosphinsäure;

3-{{N-{1-[3-(3-Amino-1,2,4-oxadiazol-5-yl)phenyl}amino}}-2(S)-hydroxy-propyl(benzyl)phosphinsäure;

3-{{N-{1-[3-(3-Amino-1,2,4-oxadiazol-5-yl)phenylamino}}-2(S)-hydroxy-propyl(cyclohexylmethyl)phosphinsäure;

3-{{N-{1-[3-(3-Chlor-1,2,4-oxadiazol-5-yl)phenylamino}}-2(S)-hydroxy-propyl(cyclohexylmethyl)phosphinsäure;

3-{{N-{1-[3-(3-Chlor-1,2,4-oxadiazol-5-yl)phenyl}amino}}-2(S)-hydroxy-propyl(benzyl)phosphinsäure;

3-{{N-{1-[4-(3-Chlor-1,2,4-oxadiazol-5-yl)phenyl}amino}}-2(S)-hydroxy-propyl(benzyl)phosphinsäure;

3-{{N-{1-[4-(3-Chlor-1,2,4-oxadiazol-5-yl)phenyl}amino}}-2(S)-hydroxy-propyl(cyclohexylmethyl)phosphinsäure;

3-{{N-{1-[4-(1,2,4-Oxadiazol-5-yl)phenyl}amino}}-2(S)-hydroxy-propyl(cyclohexylmethyl)phosphinsäure;

3-{{N-{1-[4-(1,2,4-Oxadiazol-5-yl)phenyl}amino}}-2(S)-hydroxy-propyl(benzyl)phosphinsäure;

3-{{N-{1-[3-(1,2,4-Oxadiazol-5-yl)phenyl}amino}}-2(S)-hydroxy-propyl(cyclohexylmethyl)phosphinsäure;

3-{{N-{1-[3-(1,2,4-Oxadiazol-5-yl)phenyl}amino}}-2(S)-hydroxy-propyl(benzyl)phosphinsäure;

3-{{N-[1-(3-Carboxy-4-methoxyphenyl)ethyl]amino}-2(S)-hydroxy-propyl(pyrid-3-yl-methyl)phosphinsäure;

3-{N-[1-(3-Carboxy-4-methoxyphenyl)ethyl]amino}-2(S)-hydroxy-propyl(thien-2-yl-methyl)phosphinsäure;

3-{N-[1-(3-Carboxy-4-methoxyphenyl)ethyl]amino}-2(S)-hydroxy-propyl(fur-2-yl-methyl)phosphinsäure oder jeweils ein Salz davon.

**30.** Eine Verbindung gemäss einem der Ansprüche 1 bis 29 in freier Form oder in Form eines pharmazeutische verwendbaren Salzes zur Anwendung in einem Verfahren zur Behandlung des menschlichen oder tierischen Körpers.

**31.** Ein pharmazeutisches Präparat, enthaltend eine Verbindung gemäss einem der Ansprüche 1 bis 3, 5, 9 bis 13, 15 bis 21, 23, 26, 27 und 29 in freier Form oder in Form eines pharmazeutische verwendbaren Salzes neben üblichen pharmazeutischen Hilfsstoffen.

**32.** Ein pharmazeutisches Präparat, enthaltend eine Verbindung gemäss einem der Ansprüche 2, 4, 6 bis 8, 14, 24, 25 und 28 in freier Form oder in Form eines pharmazeutische verwendbaren Salzes neben üblichen pharmazeutischen Hilfsstoffen.

**33.** Verfahren zur Herstellung neuer N-Aralkyl- und N-Heteroaralkylaminoalkanphosphinsäuren gemäss Anspruch 1 und ihrer Salze, dadurch gekennzeichnet, dass man in einer Verbindung der Formel II

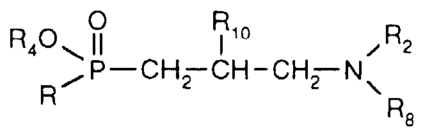

(II),

worin $R_4$ für eine Hydroxyschutzgruppe, $R_8$ für eine Gruppe $R_3$ oder für eine Aminoschutzgruppe und $R_{10}$ für Wasserstoff oder geschütztes Hydroxy steht, wobei R, $R_1$, $R_2$ und $R_3$ die angegebene Bedeutung haben, oder in einem Salz davon die Hydroxygruppen durch Ersatz der Hydroxyschutzgruppe $R_4$ durch Wasserstoff freisetzt und gegebenenfalls die Aminoschutzgruppe $R_8$ abspaltet und gegebenenfalls die geschützte Hydroxygruppen aus $R_{10}$ freisetzt und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

**34.** Eine Verbindung gemäss einem der Ansprüche 1 bis 29 zur Herstellung eines nootropen oder antiepileptischen Arzneimittels.

## Claims

**1.** A N-aralkyl- and a N-heteroaralkyl-aminoalkane-phosphinic acid of formula I

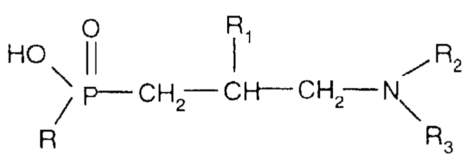

(I)

wherein R signifies $C_3$-$C_7$-alkyl, $\alpha,\alpha$-di-$C_1$-$C_4$-alkoxy-$C_1$-$C_4$-alkyl, $C_3$-$C_6$-cycloalkyl-$C_1$-$C_4$-alkyl, $C_3$-$C_6$-cycloalkenyl-$C_1$-$C_4$-alkyl, or unsubstituted phenyl-$C_1$-$C_4$-alkyl or phenyl-$C_1$-$C_4$-alkyl that is mono-, di- or trisubstituted by $C_1$-$C_4$-alkyl, $C_1$-$C_4$-alkoxy, hydroxy and/or halogen,

$R_1$ is hydrogen or hydroxy,
$R_2$ signifies a phenyl-, thienyl-, furyl- or pyridyl-$C_1$-$C_4$-alkyl radical which is mono- or disubstituted by carboxy, $C_1$-$C_4$-alkoxycarbonyl, cyano, carbamoyl, N-mono- or N,N-di-$C_1$-$C_4$-alkylcarbamoyl, carboxy-$C_1$-$C_4$-alkyl, $C_1$-$C_4$-alkoxycarbonyl-$C_1$-$C_4$-alkyl, cyano-$C_1$-$C_4$-alkyl, carbamoyl-$C_1$-$C_4$-alkyl, N-mono- or N,N-di-$C_1$-$C_4$-alkylcarbamoyl-$C_1$-$C_4$-alkyl; by oxazolyl, isoxazolyl, oxadiazolyl, triazolyl, tetrazolyl, oxazolyl-$C_1$-$C_4$-alkyl, isoxazolyl-$C_1$-$C_4$-alkyl, oxadiazolyl-$C_1$-$C_4$-alkyl, triazolyl-$C_1$-$C_4$-alkyl or tetrazolyl-$C_1$-$C_4$-alkyl, these latter being either unsubstituted or substituted by $C_1$-$C_4$-alkyl, $C_1$-$C_4$-alkoxy, $C_1$-$C_4$-alkoxycarbonyl, cyano, hydroxy, amino or halogen; the phenyl, thienyl, furyl or pyridyl-$C_1$-$C_4$-alkyl radical being optionally additionally substituted by $C_1$-$C_4$-alkoxy, polyfluoro-$C_1$-$C_4$-alkoxy, halogen or polyfluoro-$C_1$-$C_4$-alkyl, and
$R_3$ signifies hydrogen or $C_1$-$C_4$-alkyl,

or a salt thereof.

2. A compound according to claim 1 of formula I, wherein R signifies $C_2$-$C_5$-alkyl, $\alpha,\alpha$-di-$C_1$-$C_4$-alkoxymethyl, $C_3$-$C_6$-cycloalkyl-$C_1$-$C_4$-alkyl or benzyl, $R_1$ is hydrogen or hydroxy, $R_2$ signifies $\alpha,\alpha$-phenyl-$C_1$-$C_4$-alkyl radical or $\alpha$-pyridyl-$C_1$-$C_4$-alkyl radical, which is mono- or disubstituted by carboxy, $C_1$-$C_4$-alkoxycarbonyl, cyano, carbamoyl, carboxy-$C_1$-$C_4$-alkyl, $C_1$-$C_4$-alkoxycarbonyl-$C_1$-$C_4$-alkyl, cyano-$C_1$-$C_4$-alkyl, carbamoyl-$C_1$-$C_4$-alkyl, isoxazol-5-yl, isoxazol-2-yl, 1,2,4-oxadiazol-5-yl, 1,2,4-oxadiazol-3-yl, 1,3,4-oxadiazol-3-yl, 1,2,4-triazol-3-yl, 1,2,4-triazol-5-yl or tetrazol-5-yl, isoxazol-5-ylmethyl, isoxazol-2-ylmethyl, 1 ,2,4-oxadiazol-5-ylmethyl, 1,2,4-oxadiazol-3-ylmethyl, 1 , 3,4-oxadiazol-3-ylmethyl, 1 ,2,4-triazol-3-ylmethyl 1,2,4-triazol-5-ylmethyl or tetrazol-5-ylmethyl, and optionally additionally substituted by $C_1$-$C_4$-alkoxy, trifluoromethoxy, halogen, or trifluoromethyl, and $R_3$ signifies hydrogen, or a salt thereof.

3. A compound according to claim 1 of formula I, wherein R signifies $\alpha,\alpha$-di-$C_1$-$C_4$-alkoxymethyl, $C_3$-$C_6$-cycloalkyl-$C_1$-$C_4$-alkyl, $C_3$-$C_6$-cycloalk-3-enyl-$C_1$-$C_4$-alkyl or benzyl, $R_1$ is hydroxy, $R_2$ is a phenyl-$C_1$-$C_4$-alkyl radical, which is substituted by carboxy, cyano or by unsubstituted or amino- or halogen-substituted 1,2,4-oxadiazol-5-yl, and $R_3$ signifies hydrogen, or a salt thereof.

4. A compound according to claim 1 of formula I, wherein R signifies $C_3$-$C_5$-alkyl, $\alpha,\alpha$-di-$C_1$-$C_4$-alkoxymethyl, $C_3$-$C_6$-cycloalkyl-$C_1$-$C_4$-alkyl, or benzyl, $R_1$ is hydrogen or hydroxy, $R_2$ is a phenyl-$C_1$-$C_4$-alkyl radical which is mono- or di-substituted by carboxy, $C_1$-$C_4$-alkoxycarbonyl, cyano or carbamoyl, and optionally additionally substituted by $C_1$-$C_4$-alkoxy, trifluoromethoxy, halogen or trifluoromethyl, and $R_3$ signifies hydrogen, or a salt thereof.

5. A compound according to one of claims 1 to 5 of formula I, in which the C-atom of the propylene chain that is bonded to the group $R_1$, if $R_1$ is hydroxy and/or a chiral $\alpha$-C-atom of the aliphatic moiety of the radical $R_2$, if present, each have the (S) configuration.

6. 3-{N-[1-(3-cyanophenyl)ethyl]amino}-2(S)-hydroxy-propyl(cyclohexylmethyl)phosphinic acid or a salt thereof.

7. 3-{N-[1-(3-carboxyphenyl)ethyl]amino}-2(S)-hydroxy-propyl(cyclohexylmethyl)phosphinic acid or a salt thereof.

8. 3-{N-[1-(4-cyanophenyl)ethyl]amino}-2(S)-hydroxy-propyl(cyclohexylmethyl)phosphinic acid or a salt thereof.

9. 3-{N-[1(S)-(4-cyanophenyl)ethyl]amino}-2(S)-hydroxy-propyl(cyclohexylmethyl)phosphinic acid or a salt thereof.

10. 3-{N-[1(R)-(4-cyanophenyl)ethyl]amino}-2(S)-hydroxy-propyl(cyclohexylmethyl)phosphinic acid or a salt thereof.

11. 3-{N-[1(S)-(3-cyanophenyl)ethyl]amino}-2(S)-hydroxy-propyl(cyclohexylmethyl)phosphinic acid or a salt thereof.

12. 3-{N-[1 (S)-(3-cyanophenyl)ethyl]amino}-2(R)-hydroxy-propyl(cyclohexylmethyl)phosphinic acid or a salt thereof.

13. 3-{N-[1(R)-(3-cyanophenyl)ethyl]amino}-2(S)-hydroxy-propyl(cyclohexylmethyl)phosphinic acid or a salt thereof.

14. 3-{N-[1-(4-carboxyphenyl)ethyl]amino}-2(S)-hydroxy-propyl(cyclohexylmethyl)phosphinic acid or a salt thereof.

15. 3-{N-[1(S)-(4-carboxyphenyl)ethyl]amino}-2(S)-hydroxy-propyl(cyclohexylmethyl)phosphinic acid or a salt thereof.

16. 3-{N-[1(R)-(4-carboxyphenyl)ethyl]amino}-2(S)-hydroxy-propyl(cyclohexylmethyl)phosphinic acid or a salt thereof.

17. 3-{N-[1(S)-(3-carboxyphenyl)ethyl]amino}-2(S)-hydroxy-propyl(cyclohexylmethyl)phosphinic acid or a salt thereof.

18. 3-{N-[1(S)-(3-carboxyphenyl)ethyl]amino}-2(R)-hydroxy-propyl(cyclohexylmethyl)phosphinic acid or a salt thereof.

19. 3-{N-[1(R)-(3-carboxyphenyl)ethyl]amino}-2(S)-hydroxy-propyl(cyclohexylmethyl)phosphinic acid or a salt thereof.

20. 3-{N-[1(R)-(3-carboxyphenyl)ethyl]amino}-2(S)-hydroxy-propyl(cyclohex-3-enylmethyl)phosphinic acid or a salt

thereof.

**21.** 3-{N-[1(S)-(4-carboxyphenyl)ethyl]amino}-2(S)-hydroxy-propyl(cyclohex-3-enylmethyl)phosphinic acid or a salt thereof.

**22.** 3-{N-[1-(4-cyanophenyl)ethyl]amino}-2(S)-hydroxy-propyl(benzyl)phosphinic acid or a salt thereof.

**23.** 3-{N-[1-(3-cyanophenyl)ethyl]amino}-2(S)-hydroxy-propyl(benzyl)phosphinic acid or a salt thereof.

**24.** 3-{N-[1-(4-carboxyphenyl)ethyl]amino}-2(S)-hydroxy-propyl(benzyl)phosphinic acid or a salt thereof.

**25.** 3-{N-[1-(4-carboxyphenyl)ethyl]amino}-2(S)-hydroxy-propyl(benzyl)phosphinic acid or a salt thereof.

**26.** 3-{N-[1-(3-carboxyphenyl)ethyl]amino}-2(S)-hydroxy-propyl(benzyl)phosphinic acid or a salt thereof.

**27.** 3-{N-[1-(3-carboxy-4-methoxyphenyl)ethyl]amino}-2(S)-hydroxy-propyl(benzyl)phosphinic acid or a salt thereof.

**28.** 3-{{N-{1-[3-(isoxazol-5-yl)phenyl]ethyl}amino}}-2(S)-hydroxy-propyl(cyclohexylmethyl)phosphinic acid;

3-{{N-{1-[3-(isoxazol-2-yl)phenyl]ethyl}amino}}-2(S)-hydroxy-propyl(cyclohexylmethyl)phosphinic acid;

3-{{N-{1-[3-(1,2,4-oxadiazol-5-yl)phenyl]ethyl}amino}}-2(S)-hydroxy-propyl(cyclohexylmethyl)phosphinic acid;

3-{{N-{1-[3-(1,2,4-oxadiazol-3-yl)phenyl]ethyl}amino}}-2(S)-hydroxy-propyl(cyclohexylmethyl)phosphinic acid;

3-{{N-{1-[3-(1,2,4-triazol-3-yl)phenyl]ethyl}amino}}-2(S)-hydroxy-propyl(cyclohexylmethyl)phosphinic acid;

3-{{N-{1-[3-(1,2,4-triazol-5-yl)phenyl]ethyl}amino}}-2(S)-hydroxy-propyl(cyclohexylmethyl)phosphinic acid;

3-{{N-(1-[3-(tetrazol-5-yl)phenyl]ethyl}amino}}-2(S)-hydroxy-propyl(cyclohexylmethyl)phosphinic acid;

3-{{N-{1-[4-(3-amino-1,2,4-oxadiazol-5-yl)phenyl]ethyl}amino}}-2(S)-hydroxy-propyl(cyclohexylmethyl)phosphinic acid;

3-{{N-{1-[4-(3-amino-1,2,4-oxadiazol-5-yl)phenyl]ethyl}amino}}-2(S)-hydroxy-propyl(benzyl)phosphinic acid;

3-{N-[1-(4-carboxymethylphenyl)ethyl]amino}-2(S)-hydroxy-propyl(cyclohexylmethyl)phosphinic acid;

3-{N-[1-(2-carboxymethylpyrid-4-yl)ethyl]amino}-2(S)-hydroxy-propyl(cyclohexylmethyl)phosphinic acid;

3-{N-[1-(4-carboxymethylphenyl)ethyl]amino}-2(S)-hydroxy-propyl(cyclohexylmethyl)phosphinic acid, and

3-{N-[1-(4-carboxymethylphenyl)ethyl]amino}-2(S)-hydroxy-propyl(benzyl)phosphinic acid, or respectively, the salts thereof.

**29.** 3-{N-[1-(R)-(3-carboxyphenyl)ethyl]amino}-2(S)-hydroxy-propyl(diethoxymethyl)phosphinic acid;

3-{N-[1-(S)-(3-carboxyphenyl)ethyl]amino}-2(S)-hydroxy-propyl(diethoxymethyl)phosphinic acid;

3-{{N-{1-[4-(3-amino-1,2,4-oxadiazol-5-yl)phenyl}amino}}-2(S)-hydroxy-propyl(cyclohexylmethyl)phosphinic acid;

3-{{N-{1-[4-(3-amino-1,2,4-oxadiazol-5-yl)phenyl)amino}}-2(S)-hydroxy-propyl(benzyl)phosphinic acid;

3-{{N-{1-[3-(3-amino-1,2,4-oxadiazol-5-yl)phenyl}amino}}-2(S)-hydroxy-propyl(benzyl)phosphinic acid;

3-{{N-{1-[3-(3-amino-1,2,4-oxadiazol-5-yl)phenyl}amino}}-2(S)-hydroxy-propyl(cyclohexylmethyl)phosphinic acid;

3-{{N-{1-[3-(3-chloro-1,2,4-oxadiazol-5-yl)phenyl}amino}}-2(S)-hydroxy-propyl(cyclohexylmethyl)phosphinic acid;

3-{{N-{1-[3-(3-chloro-1,2,4-oxadiazol-5-yl)phenyl}amino}}-2(S)-hydroxy-propyl(benzyl)phosphinic acid;

3-{{N-{1-[4-(3-chloro-1,2,4-oxadiazol-5-yl)phenyl}amino}}-2(S)-hydroxy-propyl(benzyl)phosphinic acid;

3-{{N-{1-[4-(3-chloro-1,2,4-oxadiazol-5-yl)phenyl}amino}}-2(S)-hydroxy-propyl(cyclohexylmethyl)phosphinic acid;

3-({N-(1-[4-(1,2,4-oxadiazol-5-yl)phenyl}amino}}-2(S)-hydroxy-propyl(cyclohexylmethyl)phosphinic acid;

3-{{N-(1-[4-(1,2,4-oxadiazol-5-yl)phenyl}amino}}-2(S)-hydroxy-propyl(benzyl)phosphinic acid;

3-{{N-{1-[3-(1,2,4-oxadiazol-5-yl)phenyl}amino}}-2(S)-hydroxy-propyl(cyclohexylmethyl)phosphinic acid;

3-{{N-{1-[3-(1,2,4-oxadiazol-5-yl)phenyl}amino}}-2(S)-hydroxy-propyl(benzyl)phosphinic acid;

3-{N-[1-(3-carboxy-4-methoxyphenyl)ethyl]amino}-2(S)-hydroxy-propyl(pyrid-3-ylmethyl)phosphinic acid;

3-{N-[1-(3-carboxy-4-methoxyphenyl)ethyl]amino}-2(S)-hydroxy-propyl(thien-2-ylmethyl)phosphinic acid;

3-{N-[1-(3-carboxy-4-methoxyphenyl)ethyl]amino}-2(S)-hydroxy-propyl(fur-2-ylmethyl)phosphinic acid, or respectively, the salts thereof.

**30.** A compound according to one of claims 1 to 29 in free form or in the form of a pharmaceutically acceptable salt for use in a process for treating humans or animals.

**31.** A pharmaceutical preparation, containing a compound according to one of claims 1 to 3, 5, 9 to 13, 15 to 21, 23, 26, 27 and 29 in free form or in the form of a pharmaceutically acceptable salt, together with the usual pharmaceutical excipients.

**32.** A pharmaceutical preparation, containing a compound according to one of claims 2, 4, 6 to 8, 14, 24, 25 and 28 in free form or in the form of a pharmaceutically acceptable salt, together with the usual pharmaceutical excipients.

**33.** Process for the production of novel N-aralkyl- and N-heteroaralkyl-aminoalkane-phosphinic acids according to claim 1, and the salts thereof, characterised in that in a compound of formula II

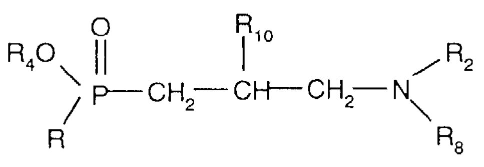

(II)

wherein $R_4$ is a hydroxy-protecting group, $R_8$ is a group $R_3$ or an amino-protecting group and $R_{10}$ is hydrogen or protected hydroxy, whereby R, $R_1$ $R_3$ and $R_3$ have the significances indicated, or in a salt thereof, the hydroxy groups are freed by replacing the hydroxy-protecting group $R_4$ with hydrogen, and where appropriate the amino-protecting group $R_8$ is cleaved, and where appropriate the protected hydroxy groups of $R_{10}$ are freed, and if desired a resulting compound is converted into another compound of formula I, an isomeric mixture obtainable according to the process is separated into its components, and the preferred isomer is separated off and/or a free compound obtainable according to the process is converted into a salt or a salt obtainable according to the process is converted into the corresponding free compound.

**34.** A compound according to one of claims 1 to 29 for the preparation of a nootropic or anti-epileptic medicament.

## Revendications

1. Un acide N-aralkyl- et N-hétéroaralkylaminoalcanephosphinique de formule I

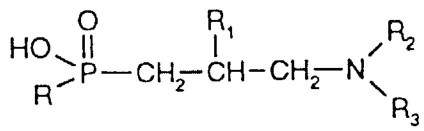

(I),

où R signifie un groupe $C_3$-$C_7$-alkyle, $\alpha,\alpha$-di-$C_1$-$C_4$-alcoxy-$C_1$-$C_4$-alkyle, $C_3$-$C_6$-cycloalkyl-$C_1$-$C_4$-alkyle, $C_3$-$C_6$-cycloalcényl-$C_1$-$C_4$-alkyle, ou un groupe phényl-$C_1$-$C_4$-alkyle non substitué ou mono-, di- ou trisubstitué par un groupe $C_1$-$C_4$-alkyle, $C_1$-$C_4$-alcoxy, hydroxy et/ou par un halogène, $R_1$ signifie l'hydrogène ou un groupe hydroxy, $R_2$ signifie un reste phényl- thiényl-, furyl- ou pyridyl-$C_1$-$C_4$-alkyle qui est mono- ou disubstitué par un groupe carboxy, $C_1$-$C_4$-alcoxycarbonyle, cyano, carbamoyle, N-mono- ou N,N-di-$C_1$-$C_4$-alkylcarbamoyle, carboxy-$C_1$-$C_4$-alkyle, $C_1$-$C_4$-alcoxycarbonyl-$C_1$-$C_4$-alkyle, cyano-$C_1$-$C_4$-alkyle, carbamoyl-$C_1$-$C_4$-alkyle, N-mono- ou N,N-di-$C_1$-$C_4$-alkylcarbamoyl-$C_1$-$C_4$-alkyle, ou mono- ou disubstitué par un groupe oxazolyle, isoxazolyle, oxadiazolyle, triazolyle, tétrazolyle, oxazolyl-$C_1$-$C_4$-alkyle, isoxazolyl-$C_1$-$C_4$-alkyle, oxadiazolyl-$C_1$-$C_4$-alkyle, triazolyl-$C_1$-$C_4$-alkyle ou tétrazolyl-$C_1$-$C_4$-alkyle, non substitué ou substitué par un groupe $C_1$-$C_4$-alkyle, $C_1$-$C_4$-alcoxy, $C_1$-$C_4$-alcoxycarbonyle, cyano, hydroxy, amino ou un halogène, et éventuellement en outre substitué par un groupe $C_1$-$C_4$-alcoxy, polyfluoro-$C_1$-$C_4$-alcoxy, un halogène ou un groupe polyfluoro-$C_1$-$C_4$-alkyl , et $R_3$ signifie l'hydrogène ou un groupe $C_1$-$C_4$-alkyle, ou un de ses sels.

2. Un composé selon la revendication 1 de formule I, où R signifie un groupe $C_3$-$C_5$-alkyle, $\alpha,\alpha$-di-$C_1$-$C_4$-alcoxyméthyle, $C_3$-$C_6$-cycloalkyl-$C_1$-$C_4$-alkyle ou benzyle, $R_1$ signifie l'hydrogène ou un groupe hydroxy, $R_2$ signifie un reste $\alpha$-phényl-$C_1$-$C_4$alkyle ou $\alpha$-pyridyl-$C_1$-$C_4$alkyle mono ou disubstitué par un groupe carboxy, $C_1$-$C_4$-alcoxycarbonyle, cyano, carbamoyle, carboxy-$C_1$-$C_4$-alkyle, $C_1$-$C_4$-alcoxycarbonyl-$C_1$-$C_4$alkyle, cyano-$C_1$-$C_4$-alkyle, carbamoyl-$C_1$-$C_4$-alkyle, isoxazol-5-yle, isoxazol-2-yle, 1,2,4-oxadiazol-5-yle, 1,2,4-oxadiazol-3-yle, 1,3,4-oxadiazol-3-yle, 1,2,4-triazol-3-yle, 1,2,4-triazol-5-yle ou tétrazol-5-yle, isoxazol-5-ylméthyle, isoxazol-2-ylméthyle, 1,2,4-oxadiazol-5-ylméthyle, 1,2,4-oxadiazol-3-ylméthyle, 1,3,4-oxadiazol-3-ylméthyle, 1,2,4-triazol-3-ylméthyle, 1,2,4-triazol-5-ylméthyle ou tétrazol-5-ylméthyle, et éventuellement substitué en outre par un groupe $C_1$-$C_4$-alcoxy, trifluorométhoxy, un halogène ou un groupe trifluorométhyle et $R_3$ signifie l'hydrogène, ou un de ses sels.

3. Un composé selon la revendication 1 de formule I, où R signifie un groupe $\alpha,\alpha$-di-$C_1$-$C_4$-alcoxyméthyle, $C_3$-$C_6$-cycloalkyl-$C_1$-$C_4$-alkyle, $C_3$-$C_6$-cycloalk-3-ényl-$C_1$-$C_4$-alkyle ou benzyle, $R_1$ signifie un groupe hydroxy, $R_2$ signifie un reste phényl-$C_1$-$C_4$alkyle substitué par un groupe carboxy, cyano ou par un groupe 1,2,4-oxadiazol-5-yle non substitué ou substitué par un groupe amino ou un halogène et $R_3$ signifie l'hydrogène, ou un de ses sels.

4. Un composé selon la revendication 1 de formule I, où R signifie un groupe $C_3$-$C_5$-alkyle, $\alpha,\alpha$-di-$C_1$-$C_4$-alcoxyméthyle, $C_3$-$C_6$-cycloalkyl-$C_1$-$C_4$-alkyle ou benzyle, $R_1$ signifie l'hydrogène ou un groupe hydroxy, $R_2$ signifie un reste phényl-$C_1$-$C_4$-alkyle mono- ou di-substitué par un groupe carboxy, $C_1$-$C_4$-alcoxycarbonyle, cyano ou carbamoyle et éventuellement substitué en outre par un groupe $C_1$-$C_4$-alcoxy, trifluorométhoxy, un halogène ou un groupe trifluorométhyle et $R_3$ signifie l'hydrogène ou un de ses sels.

5. Un composé selon l'une des revendications 1 à 5 de formule I, dans lequel l'atome de carbone de la chaîne propylène lié au groupe $R_1$, lorsque $R_1$ signifie un groupe hydroxy et/ou un atome de carbone $\alpha$ chiral de la partie aliphatique du reste $R_2$, lorsqu'il est présent, ont chacun la configuration (S).

6. L'acide 3-{N-[1-(3-cyanophényl)éthyl]amino}-2(S)-hydroxy-propyl(cyclohexylméthyl)phosphinique ou un de ses sels.

7. L'acide 3-{N-[1-(3-carboxyphényl)éthyl]amino}-2(S)-hydroxy-propyl(cyclohexylméthyl)phosphinique ou un de ses sels.

8. L'acide 3-{N-[1-(4-cyanophényl)éthyl]amino}-2(S)-hydroxy-propy](cyclohexylméthyl)phosphinique ou un de ses sels.

**9.** L'acide 3-{N-[1(S)-(4-cyanophényl)éthyl]amino}-2(S)-hydroxy-propyl(cyclohexylméthyl)phosphinique ou un de ses sels.

**10.** L'acide 3-{N-[1(R)-(4-cyanophényl)éthyl]amino}-2(S)-hydroxy-propyl(cyclohexylméthyl)phosphinique ou un de ses sels.

**11.** L'acide 3-{N-[1(S)-(3-cyanophényl)éthyl]amino}-2(S)-hydroxy-propyl(cyclohexylméthyl)phosphinique ou un de ses sels.

**12.** L'acide 3-{N-[1(S)-(3-cyanophényl)éthyl]amino}-2(R)-hydroxy-propyl(cyclohexylméthyl)phosphinique ou un de ses sels.

**13.** L'acide 3-{N-[1(R)-(3-cyanophényl)éthyl}amino}-2(S)-hydroxy-propyl(cyclohexylméthyl)phosphinique ou un de ses sels.

**14.** L'acide 3-{N-[1-(4-carboxyphényl)éthyl]amino}-2(S)-hydroxy-propyl(cyclohexylméthyl)phosphinique ou un de ses sels.

**15.** L'acide 3-{N-[1(S)-(4-carboxyphényl)éthy]]amino}-2(S)-hydroxypropyl(cyclohexylrnéthyl)phosphinique ou un de ses sels.

**16.** L'acide 3-{N-[1(R)-(4-carboxyphényl)éthyl]amino}-2(S)-hydroxypropyl(cyclohexylméthyl)phosphinique ou un de ses sels.

**17.** L'acide 3-{N-[1(S)-(3-carboxyphényl)éthyl]amino}-2(S)-hydroxypropyl(cyclohexylméthyl)phosphinique ou un de ses sels.

**18.** L'acide 3-{N-[1(S)-(3-carboxyphényl)éthyl]amino}-2(R)-hydroxypropyl(cyclohexylméthyl)phosphinique ou un de ses sels.

**19.** L'acide 3-{N-[1(R)-(3-carboxyphényl)éthyl]amino}-2(S)-hydroxypropyl(cyclohexylméthyl)phosphinique ou un de ses sels.

**20.** L'acide 3-{N-[1(R)-(3-carboxyphényl)éthyl]amino}-2(S)-hydroxypropyl(cyclohex-3-énylméthyl)phosphinique ou un de ses sels.

**21.** L'acide 3-{N-[1(S)-(4-carboxyphényl)éthyl]amino}-2(S)-hydroxypropyl(cyclohex-3-énylméthyl)phosphinique ou un de ses sels.

**22.** L'acide 3-{N-[1-(4-cyanophényl)éthyl]amino}-2(S)-hydroxy-propyl(benzyl)phosphinique ou un de ses sels.

**23.** L'acide 3-{N-[1-(3-cyanophényl)éthyl]amino}-2(S)-hydroxy-propyl(benzyl)phosphinique ou un de ses sels.

**24.** L'acide 3-{N-[1-(4-carboxyphényl)éthyl]amino}-2(S)-hydroxy-propyl(benzyl)phosphinique ou un de ses sels.

**25.** L'acide 3-{N-[1-(4-carboxyphény])éthyl]amino}-2(S)-hydroxy-propyl(benzyl)phosphinique ou un de ses sels.

**26.** L'acide 3-{N-[1-(3-carboxyphényl)éthyl]amino}-2(S)-hydroxy-propyl(benzyl)phosphinique ou un de ses sels.

**27.** L'acide 3-{N-[1-(3-carboxy-4-méthoxyphényl)éthyl]amino}-2(S)-hydroxy-propyl(benzyl)phosphinique ou un de ses sels.

**28.** L'acide 3-{{N-{1-[3-(isoxazol-5-yl)phényl]éthyl}amino}}-2(S)-hydroxy-propyl(cyclohexylméthyl)phosphinique;

L'acide 3-{{N-(1-[3-(isoxazol-2-yl)phényl]éthyl}amino}}-2(S)-hydroxy-propyl(cyclohexylméthyl)phosphinique;

L'acide 3-{{N-{1-[3-(1,2,4-oxadiazol-5-yl)phényl]éthyl}amino}}-2(S)-hydroxypropyl(cyclohexylméthyl)phosphinique;

L'acide 3-{{N-{1-[3-(1,2,4-oxadiazol-3-yl)phényl]éthyl}amino}}-2(S)-hydroxypropyl(cyclohexylméthyl)phosphinique;

L'acide 3-{{N-{1-[3-(1,2,4-triazol-3-yl)phényl]éthyl}amino}}-2(S)-hydroxy-propyl(cyclohexylméthyl)phosphinique;

L'acide 3-{{N-{1-[3-(1,2,4-triazol-5-yl)phényl]éthyl}amino}}-2(S)-hydroxy-propyl(cyclohexylméthyl)phosphinique;

L'acide 3-{{N-{1-[3-(tétrazol-5-yl)phényl]ethyl}amino}}-2(S)-hydroxy-propyl(cyclohexylméthyl)phosphinique;

L'acide 3-{{N-{1-[4-(3-amino-1,2,4-oxadiazol-5-yl)phényl]éthyl}amino}}-2(S)-hydroxy-propyl(cyclohexylméthyl)phosphinique;

L'acide 3-{{N-{1-[4-(3-amino-1,2,4-oxadiazol-5-yl)phényl]éthyl}amino}}-2(S)-hydroxy-propyl(benzyl)phosphinique;

L'acide 3-{N-1-(4-(4-carboxyméthylphényl)éthyl]amino}-2(S)-hydroxy-propyl(cyclohexylméthyl)phosphinique;

L'acide 3-{N-[1-(2-carboxyméthylpyrid-4-yl)éthyl]amino}-2(S)-hydroxy-propyl(cyclohexylméthyl)phosphinique;

L'acide 3-{N-[1-(4-carboxyméthylphényl)éthyl]amino}-2(S)-hydroxy-propyl(cyclohexylméthyl)phosphinique ou

L'acide 3-{N-[1-(4-carboxyméthylphényl)éthyl]amino}-2(S)-hydroxy-propyl(benzyl)phosphinique ou un de ses sels.

**29.** L'acide 3-{N-[1-(R)-(3-carboxyphényl)éthyl]amino}-2(S)-hydroxypropyl(diéthoxyméthyl)phosphinique;

L'acide 3-{N-[1-(S)-(3-carboxyphényl)éthyl]amino}-2(S)-hydroxy-propyl(diéthoxyméthyl)phosphinique;

L'acide 3-{{N-{1-[4-(3-amino-1,2,4-oxadiazol-5-yl)phényl}amino}}-2(S)-hydroxypropyl(cyclohexylméthyl) phosphinique;

L'acide 3-{{N-{1-[4-(3-amino-1,2,4-oxadiazol-5-yl)phényl}amino}}-2(S)-hydroxypropyl(benzyl)phosphinique;

L'acide 3-{{N-{1-[3-(3-amino-1,2,4-oxadiazol-5-yl)phényl}amino}}-2(S)-hydroxypropyl(benzyl)phosphinique;

L'acide 3-{{N-{1-[3-(3-amino-1,2,4-oxadiazol-5-yl)phényl}amino}}-2(S)-hydroxypropyl(cyclohexylméthyl) phosphinique;

L'acide 3-{{N-{1-[3-(3-chloro-1,2,4-oxadiazol-5-yl)phényl}amino}}-2(S)-hydroxypropyl(cyclohexylméthyl) phosphinique;

L'acide 3-{{N-{1-[3-(3-chloro-1,2,4-oxadiazol-5-yl)phényl}amino}}-2(S)-hydroxypropyl(benzyl)phosphinique;

L'acide 3-{{N-{1-[4-(3-chloro-1,2,4-oxadiazol-5-yl)phényl}amino}}-2(S)-hydroxypropyl(benzyl)phosphinique;

L'acide 3-{{N-{1-[4-(3-chloro-1,2,4-oxadiazol-5-yl)phényl}amino}}-2(S)-hydroxypropyl(cyclohexylméthyl) phosphinique;

L'acide 3-{{N-{1-[4-(1,2,4-oxadiazol-5-yl)phényl}amino}}-2(S)-hydroxy-propyl(cyclohexylméthyl)phosphinique;

L'acide 3-{{N-{1-[4-(1,2,4-oxadiazol-5-yl)phényl}amino}}-2(S)-hydroxy-propyl(benzyl)phosphinique;

L'acide 3-{{N-{1-[3-(1,2,4-oxadiazol-5-yl)phényl}amino}}-2(S)-hydroxy-propyl(cyclohexylméthyl)phosphinique;

L'acide 3-{{N-{1-[3-(1,2,4-oxadiazol-5-yl)phényl}amino}}-2(S)-hydroxy-propyl(benzyl)phosphinique;

L'acide 3-{N-[1-(3-carboxy-4-méthoxyphényl)éthyl]amino}-2(S)-hydroxy-propyl(pyridyl-3-méthyl)phosphinique;

L'acide 3-{N-[1-(3-carboxy-4-méthoxyphényl)éthyl]amino}-2(S)-hydroxy-propyl(thiényl-2-méthyl)phosphinique;

L'acide 3-{N-[1-(3-carboxy-4-méthoxyphényl)éthyl]amino}-2(S)-hydroxy-propyl(furyl-2-méthyl)phosphinique ou l'un de ses sels.

**30.** Un composé selon l'une des revendications 1 à 29, sous forme libre ou sous forme d'un sel pharmaceutiquement acceptable pour une utilisation dans un procédé de traitement du corps humain ou animal.

**31.** Une composition pharmaceutique contenant un composé selon l'une des revendications 1 à 3, 5, 9 à 13, 15 à 21, 23, 26, 27 et 29, sous forme libre ou sous forme d'un sel pharmaceutiquement acceptable, outre les excipients pharmaceutiques habituels.

**32.** Une composition pharmaceutique, contenant un composé selon l'une des revendications 2, 4, 6 à 8, 14, 24, 25 et 28, sous forme libre ou sous forme d'un sel pharmaceutiquement acceptable, outre les excipients pharmaceutiques habituels.

**33.** Un procédé de préparation des nouveaux acides N-aralkyl- et N-hétéroaralkylaminoalcanephosphiniques selon la revendication 1 et de leurs sels, caractérisé en ce que dans un composé de formule II

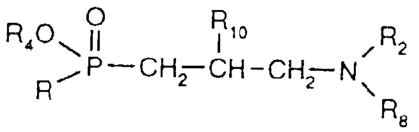

(II),

où $R_4$ signifie un groupe protecteur du groupe hydroxy, $R_8$ signifie un groupe $R_3$ ou signifie un groupe protecteur du groupe amino et $R_{10}$ signifie l'hydrogène ou un groupe hydroxy protégé, R, $R_1$, $R_2$ et $R_3$ ayant la signification indiquée, ou dans un de ses sels, on libère les groupes hydroxy en remplaçant le groupe protecteur du groupe hydroxy $R_4$ par de l'hydrogène et on élimine éventuellement le groupe protecteur du groupe amino $R_8$ et on libère éventuellement le groupe hydroxy protégé $R_{10}$ et on transforme éventuellement un composé obtenu en un autre composé de formule I, on sépare un mélange d'isomères obtenu selon le procédé en composants et on sépare à chaque fois les isomères préférés et/ou on transforme un composé libre obtenu selon le procédé en un sel ou un sel obtenu selon le procédé en un composé libre correspondant.

**34.** Un composé selon l'une des revendications 1 à 29, pour la préparation d'un médicament nootropique ou antiépileptique.